(19) **Europäisches Patentamt**
**European Patent Office**
**Office européen des brevets**

(11) **EP 1 468 392 B1**

(12) **EUROPEAN PATENT SPECIFICATION**

(45) Date of publication and mention
of the grant of the patent:
**04.04.2007 Bulletin 2007/14**

(51) Int Cl.:
***G06F 19/00*** *(2006.01)*

(21) Application number: **03700921.4**

(86) International application number:
**PCT/GB2003/000241**

(22) Date of filing: **20.01.2003**

(87) International publication number:
**WO 2003/062468 (31.07.2003 Gazette 2003/31)**

(54) **METHOD FOR BINDING SITE IDENTIFICATION USING A MULTI-SCALE APPROACH**

VERFAHREN ZUR IDENTIFIZIERUNG VON BINDUNGSSTELLEN MIT EINEM "MULTI-SCALE APPROACH"

METHODE D'IDENTIFICATION DE SITES DE LIAISON UTILISANT UNE APPROCHE "MULTI-ECHELLE"

(84) Designated Contracting States:
**AT BE BG CH CY CZ DE DK EE ES FI FR GB GR HU IE IT LI LU MC NL PT SE SI SK TR**

(30) Priority: **25.01.2002 GB 0201754**

(43) Date of publication of application:
**20.10.2004 Bulletin 2004/43**

(73) Proprietor: **ISIS INNOVATION LIMITED**
**Summertown,**
**Oxford OX2 7SG (GB)**

(72) Inventors:
• **GLICK, Meir**
**East Hanover, NJ 07936-1080 (US)**
• **RICHARDS, William Graham,**
**University of Oxford**
**Oxford OX1 3QH (GB)**
• **ROBINSON, Daniel D.**
**Towcester,**
**Northamptonshire N12 6EA (GB)**

(74) Representative: **Tyson, Robin Edward**
**J.A. Kemp & Co.,**
**14 South Square,**
**Gray's Inn**
**London WC1R 5JJ (GB)**

(56) References cited:

• **GLICK MEIR ET AL: "Identification of ligand binding sites on proteins using a multi-scale approach." JOURNAL OF THE AMERICAN CHEMICAL SOCIETY, vol. 124, no. 10, 13 March 2002 (2002-03-13), pages 2337-2344, XP002243466 March 13, 2002 ISSN: 0002-7863**
• **GLICK MEIR ET AL: "Docking of flexible molecules using multiscale ligand representations." JOURNAL OF MEDICINAL CHEMISTRY, vol. 45, no. 21, 10 October 2002 (2002-10-10), pages 4639-4646, XP002243467 October 10, 2002 ISSN: 0022-2623**
• **DAVIES E KEITH ET AL: "Pattern recognition and massively distributed computing." JOURNAL OF COMPUTATIONAL CHEMISTRY. UNITED STATES DEC 2002, vol. 23, no. 16, December 2002 (2002-12), pages 1544-1550, XP002243468 ISSN: 0192-8651**
• **WADE REBECCA C ET AL: "Further development of hydrogen bond functions for use in determining energetically favorable binding sites on molecules of known structure: 1. Ligand probe groups with the ability to form two hydrogen bonds." JOURNAL OF MEDICINAL CHEMISTRY, vol. 36, no. 1, 1993, pages 140-147, XP002243469 ISSN: 0022-2623**
• **WADE REBECCA C ET AL: "Further development of hydrogen bond functions for use in determining energetically favorable binding sites on molecules of known structure: 2. Ligand probe groups with the ability to form more than two hydrogen bonds." JOURNAL OF MEDICINAL CHEMISTRY, vol. 36, no. 1, 1993, pages 148-156, XP002243470 ISSN: 0022-2623**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 468 392 B1

**EP 1 468 392 B1**

## Description

The present invention relates to a method of molecular recognition, in particular for identifying a binding site for a ligand on a macromolecule and the configuration of the ligand with respect to the macromolecule when bound.

Genomics, proteomics and bioinformatics are yielding novel therapeutic targets for drug discovery efforts at a rapid rate. The genome projects reveal a plethora of new sequences. Their 3D structures will be solved by various techniques such as X-ray crystallography or Nuclear Magnetic Resonance (NMR). Even once detailed structures are known, the design of candidate drugs that may interact with these targets is a difficult task. Novel theoretical approaches will become a major avenue for drug discovery efforts and improve our ability to deal with the abundance of information at the post-genomic era. Docking algorithms are essential in rational drug design. Previously it has been known to search amongst a virtual library of billions of small molecules for compounds that can bind to known protein binding sites. In such circumstances, a matching algorithm such as DOCK, which employs spheres to model the binding site, or THINK, which orients the ligand towards chemically favorable interactions zones, or FLEXX, which is an incremental construction method, can rapidly dock the potential ligand. However, it is necessary to provide *a priori* knowledge of the binding site.

Let us assume the ligand and host molecules are rigid. Regrettably, a brute-force search, where the ligand-host interaction energy is evaluated at all possible docking configurations, cannot be completed in a reasonable amount of computing time, particularly when employing a large protein or ligand. To gain some idea of the runtime requirements we might consider that a typical protein host might occupy a volume of some $60Å^3$. Even with a moderate translational resolution of 1Å this leaves 216000 translations to search. Given a reasonable rotational resolution of 20° in each axis, and given that a potential ligand and protein might contain 35 atoms and 3500 atoms respectively, $1.5*10^{14}$ pairwise nonbonding energy evaluations will be needed to scan the complete range of possible docking configurations. Even if one can evict 99% of the points by employing various assumptions, we will still require $1.5*10^{12}$ evaluations. As a result, brute force approaches such as GRID are limited to small probe groups and cannot handle a detailed ligand in a reasonable computing time. One problem of ligand-host docking is to reduce the number of energy evaluations that need to be performed in order to locate the optimum binding position.

A different approach is based on a docking simulation in a given potential field via a global optimization algorithm that minimizes the ligand protein interaction energy: methods such as Monte Carlo simulated annealing, genetic algorithms or the multiple copy simultaneous search (MCSS) method have proved successful in this respect. In general, these methodologies give reasonable results provided the initial position of the ligand is within, or proximate to, the binding site of the host molecule. By starting the simulation in a good position we can restrict the number of docking configurations that need to be considered and avoid many of the local entrapments. However, there still exists the problem that failure to assist the optimizer in this manner will result in an extremely long calculation that might fail to detect the global minimum.

Some virtual screening methods take into account ligand flexibility. The geometry and the estimated binding free-energy of protein-ligand complexes are calculated for each conformer. However, one problem with this strategy is that it consumes vast computing time and disk space during high throughput docking (HTD) of millions or even billions of molecules.

It is an object of the present invention to alleviate, at least partially, the above problems.

Accordingly, the present invention provides a computer-based method for identifying a binding site for a ligand on a macromolecule and the configuration of the ligand when bound, said method comprising using a multiscale approach to represent the ligand as a plurality of models having different levels of detail.

The invention enables a binding site to be identified in a computationally feasible amount of time without any *a priori* knowledge. Preferably, using said multiscale approach comprises the steps of:

representing the ligand as a model comprising number of feature points fewer than the number of atoms in the ligand,

evaluating the interaction energy between the ligand model and the macromolecule for a plurality of spatial positions and/or orientations of the ligand model, as represented by the feature points, relative to the macromolecule;

eliminating those spatial positions and/or orientations for which the interaction energy does not satisfy a predetermined criterion;

iterating the above steps using an increased number of feature points for the ligand model at each successive iteration.

In a brute force approach numerous operations are redundant since in many configurations the ligand atoms will be either too distant or close to the protein atoms. This invention uses the realisation that one can eliminate these configurations at an early stage by employing a filtering operation thus saving a substantial amount of computing time. This strategy is adapted from methods developed in the fields of signal and image processing: the so-called *multi-scale approach* (see Forgy, E. *Biometrics,* **1965**, *21*, 768-769, and MacQueen, J. In *Proceedings of the fifth Berkeley Symposium on Mathematical Statistics and Probability. Volume I, Statistics;* Le Cam, L.E.; Neyman, J, Eds.; University of California Press, 1967). The invention is based upon the multi-scale concept where one deals with a hierarchy of models for the potential ligand. This is done by a simple approach whereby a potential ligand is represented by a growing number of feature points. At each increasing level of detail, a pruning of potential binding sites is performed. Preferably a nonbonding energy function is used to score the interactions between molecules at each step. Applying this approach enables one to return to a simpler, rapid brute-force style search, removing the need for an elaborate optimization protocol.

Preferably the ligand is represented as a single feature point for the first iteration which has the advantage of greatly increasing the speed of the first iteration since there are no orientations to consider.

Preferably the number of feature points in the model representing the ligand is increased by one at each iteration. This means that for the second iteration having only two feature points there are only two axes about which rotation of the ligand needs to be considered, which again reduces computation time.

Preferably said predetermined criterion in said eliminating step is one of:

that the interaction energy is below a threshold value, such as zero;
that the interaction energy is ranked in the lowest predetermined number of all the energies for that particular iteration, such as the lowest 5; or
that the interaction energy is ranked in the lowest predetermined fraction of all the energies for that particular iteration, such as the lowest 10%.

Advantageously the invention preferably comprises applying a k-means-clustering algorithm to represent the ligand as a model.

Preferably the models of the ligand comprise averages of the atomic parameters of the ligand, said parameters comprising the spatial position of the atoms and at least one atomic force field descriptor. This has the advantage that it is computationally simple to calculate the averages of the atomic parameters for each cluster represented by a feature point. Preferably said at least one atomic force field descriptor comprises at least one selected from the group consisting of: atom type, charge and polarizability. It is straightforward to increase the sophistication of the model by adding more descriptors to be used in the interaction energy evaluation.

Preferably the invention further provides terminating the iterative process after one of:

a predetermined number of iterations;
the number of non-eliminated configurations of the ligand model is smaller than a predetermined number;
the distribution of non-eliminated configurations of the ligand model is within a predetermined range..

Preferably the method of the invention further comprises determining the optimal binding site and ligand configuration by: selecting either that configuration model with the lowest energy or the average configuration of a predetermined number of lowest ranked configuration models; and reconstructing the ligand configuration from the selected model.

Optionally the method of the invention further comprises applying a local energy minimization to determine an optimized binding site and ligand configuration. The multi-scale approach can be used to identify the potential binding site rapidly, perhaps in only four iterations, and then a more precise determination can be performed using other optimization techniques.

Preferably the method of the invention further comprises the step of:

initially identifying a representative set of conformations of the ligand.

Preferably the representative set of conformations of the ligand is obtained by rotating each rotatable bond of the ligand through a predetermined angular interval.

Preferably the predetermined angular interval is greater than 10°, more preferably greater than 20°, most preferably at least 30°, such as 45° or even 60°. This greatly reduces the computational burden.

Preferably, if the number of conformations exceeds a predetermined quantity, then that quantity of conformers are sampled at random.

Preferably the method further comprises the steps of:

evaluating the molecular energy of each identified conformation; and
eliminating all conformations whose energy exceeds a predetermined amount, for example approximately 12 Kcal/mol.

Optionally, the method further comprises the steps of, at each iteration:

representing each remaining ligand conformation as a model comprising a number of feature points; and

applying a purge criterion to the model representations to obtain a smaller number of models for evaluating their interaction energy with the macromolecule.

Purging is efficient at reducing the number of potential models to be docked.

Preferably the purge criterion comprises: treating as a single model those models for which the variation in distance between particular feature points is less than 1.3 Å, and the variation in angle between particular arms of the model is less than 30° (for models with 3 or more points), and the variation in a particular dihedral angle is less than 30° (for models with 4 or more points).

Preferably the method further comprises the step of, when increasing the number of feature points for the ligand model, only considering those ligand conformations represented by the model from the preceding iteration which had the lowest interaction energy with the macromolecule. This enables an efficient search tree to be constructed.

Preferably the invention further comprises outputting, in digital form, information representing the identified binding site and/or ligand configuration and/or conformation when bound.

A further aspect of the invention provides a method comprising using binding site and/or ligand configuration and/or conformation information obtained according to any above method to design mutations of the macromolecule and/or variations in the ligand which either agonise or antagonise the binding of the ligand at the binding site.

A further aspect of the invention provides a method of identifying pharmaceutically useful compounds comprising the steps of:

repeating the any method of the invention as outlined above for a plurality of different ligands separately on the same macromolecule;
comparing the identified binding site for each ligand with a known binding site; and
identifying as useful compounds those ligands for which the identified binding site matches the known binding site.

A further aspect of the invention provides a system for determining a binding site for a ligand on a macromolecule and the configuration of the ligand when bound, comprising:

interface means adapted to receive atomic information on the ligand and macromolecule;
a module comprising means to use a multiscale approach to represent the ligand as a plurality of models having different levels of detail and means to perform interaction energy evaluations to eliminate unfavourable binding sites and configurations; and
interface means adapted to output information on the or each determined binding site and ligand configuration.

Preferably the system is arranged to perform the method of the invention as defined above.

The invention also provides a computer program comprising code means which, when executed by a data processing system, performs all the method steps of a method according to the invention as defined above.

Methods embodying the invention can be implemented as computer software comprising computer instructions or code, stored on a computer-readable storage medium. The invention can be embodied as a system for executing such software, using a computer processor. The system includes interface means adapted to receive ligand and macromolecule information, for example from a user interface, or from a storage medium, or over a communications network, such as the Internet. The system also comprises a module comprising means to perform a method embodying the invention. The system includes interface means adapted to output resulting binding site information and/or ligand configuration and/or conformation information, for example by displaying it on a screen, printing it, storing it on a storage medium or sending it over a communications network.

Embodiments of the invention will now be described, by way of example only, with reference to the accompanying drawings in which:-

**Figure 1** depicts examples of models generated for the HIV-Reverse Transcriptase inhibitor nevirapine;
**Figure 2** illustrates chemical formulae for nine ligands selected as test cases in the first set of example results;
**Figure 3** gives comparison between the configurations of the ligand as predicted (shown as dark) and the known crystal structure (atoms of oxygen, nitrogen, sulphur and phosphorus are labeled O, N, S and P, respectively; other unlabelled atoms are carbon, and hydrogen atoms are omitted for clarity). The protein is shown as a ribbon.

**a**. Streptavidin / biotin. H-bonds shown by dashed lines.
**b**. McPC-603 / phosphocholine results.
**c**. β-Trypsin/benzamidine
**d**. Cytochrome P-450 cam/camphor;

**Figure 4** illustrates search results for HIV-Reverse Transcriptase. Lowest energy grid points (shown as light grey dots) (up to 20,000 points). The protein is shown as a dark grey ribbon.

    **a**. first iteration for nevirapine

    **b**. third iteration for nevirapine

    **c**. last iteration for nevirapine (crystal structure configuration is shown as a stick model on the left, with the lowest energy predicted positions as grey spheres)

    **d.** The configuration of the predicted nevirapine configuration is shown as the light grey stick model, the configuration of the ligand in the crystal structure is shown as the dark stick model (the lowest energy grid points are shown as spheres).

    **e.** The interactions between various HIV-Reverse Transcriptase inhibitors in their predicted position and the active site's residues. Nevirapine is shown in dark grey; 1051U91 is shown in light grey; $\alpha$-APA is shown in mid grey, and the active site residues are shown around the ligands. Hydrogens are not shown.

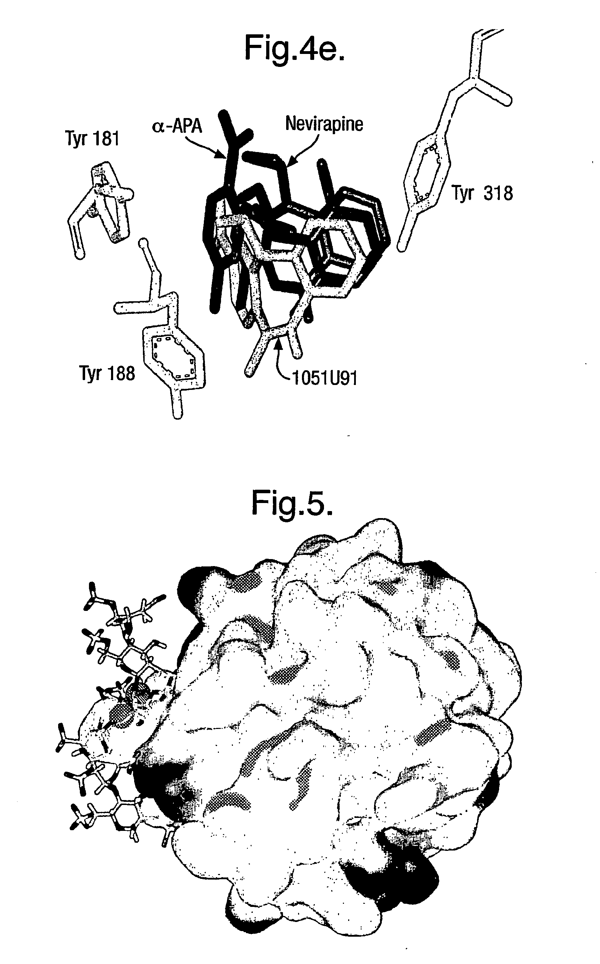

**Figure 5** illustrates search results on heparin (shown as a stick model)-basic fibroblast growth factor (bFGF). The surface of bFGF is shaded to indicate electrostatic potential (stippling indicating negative electrostatic potential and dark shaded regions having positive electrostatic potential). The cross-hatched sphere on the left indicates the centroid of the ligand in crystal structure, and the other spheres on the left are five low energy configurations).

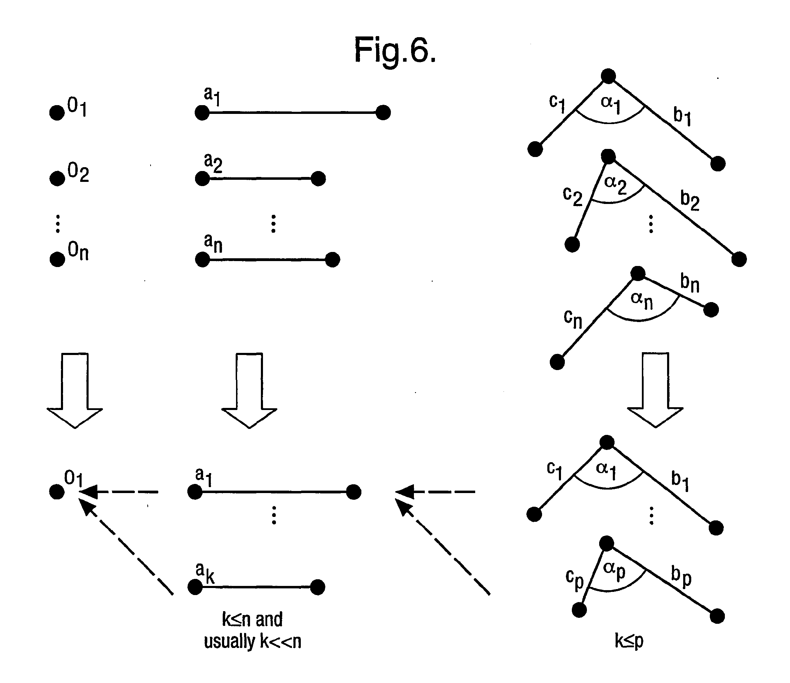

**Figure 6** depicts *k-means* clustering results for an increasing number of feature points (from 1 to 3) for n conformers. In a single point representation, all the conformers'. representations ($O_1$, $O_2$...$O_n$) are identical and can be purged into a single point. The two points cluster is defined as a line ($a_1$, $a_2$...$a_n$) and three points cluster as two lines and an angle ($b_1$, $b_2$...$b_n$), ($c_1$, $c_2$...$c_n$), ($\alpha_1$, $\alpha_2$...$\alpha_n$). All these clusters can still be purged into a much smaller number than n, either k for the two points or p for the three points.

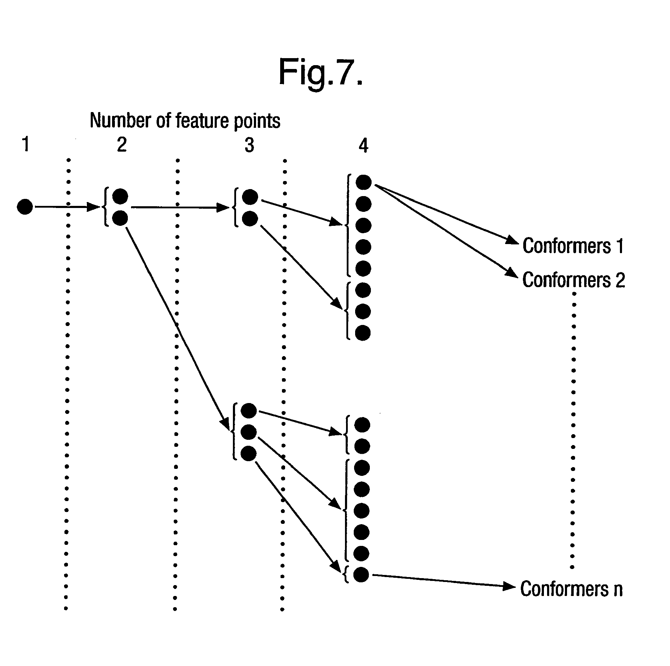

**Figure 7** illustrates how the purged clusters are sorted to form a search tree. The search begins from a single point purged cluster. Then it moves to the lower energy two point purged cluster, three point and four. The search is done only on the relevant branches.

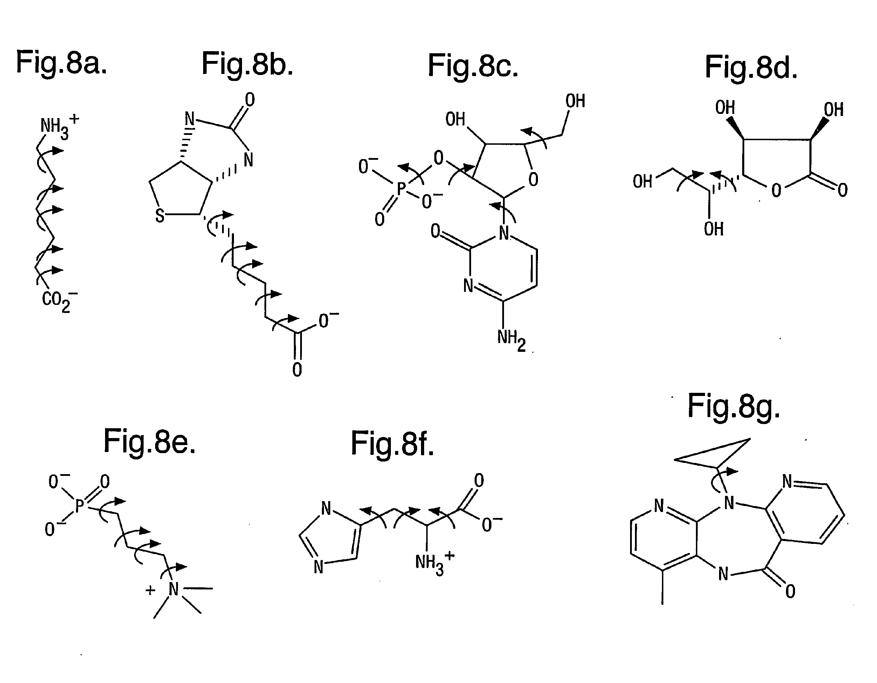

**Figure 8** illustrates chemical formulae for seven ligands used as test cases in the further example results: (a) $\varepsilon$-amino capronic acid, (b) biotin, (c) cytidylic acid, (d) L-ascorbic acid, (e) phosphocholine, (f) L-histidine, (g) nevirapin. The rotations are shown with arrows. Methyl moieties are not rotated.

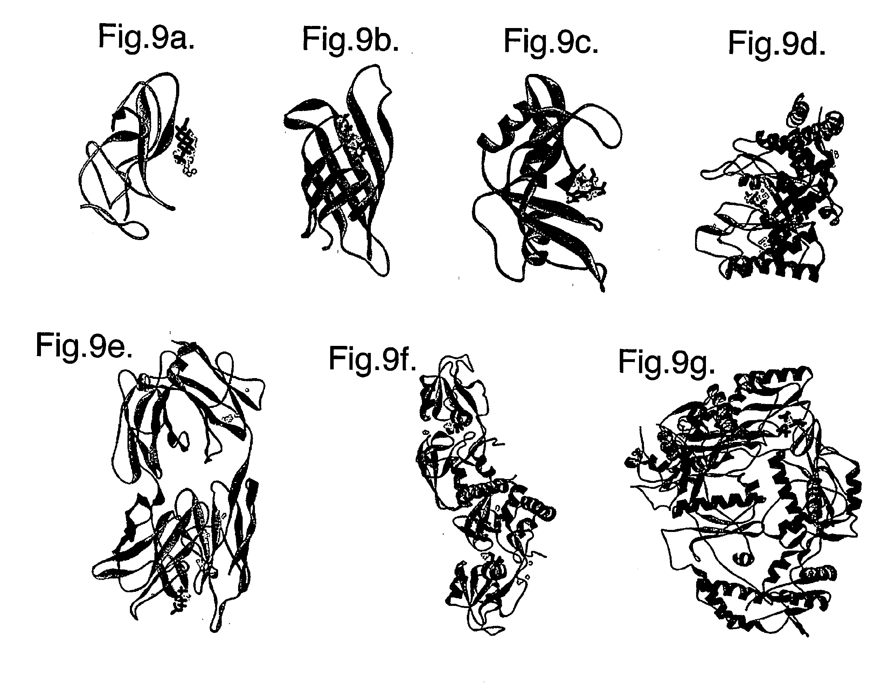

**Figure 9** illustrates the seven proteins chosen as test cases corresponding to the seven ligands of Figure 8: (a) hydrolase (serine protease), (b) streptavidin, (c) hydrolase (endoribonuclease), (d) isomerase, (e) McPC-603, (f) histidine binding protein, (g) HIV reverse transcriptase. The proteins are shown as ribbons. The ligand in the crystal structure is shown as a dark stick model. The remaining possible translations of the ligand at the end of the third iteration are shown as light grey spheres

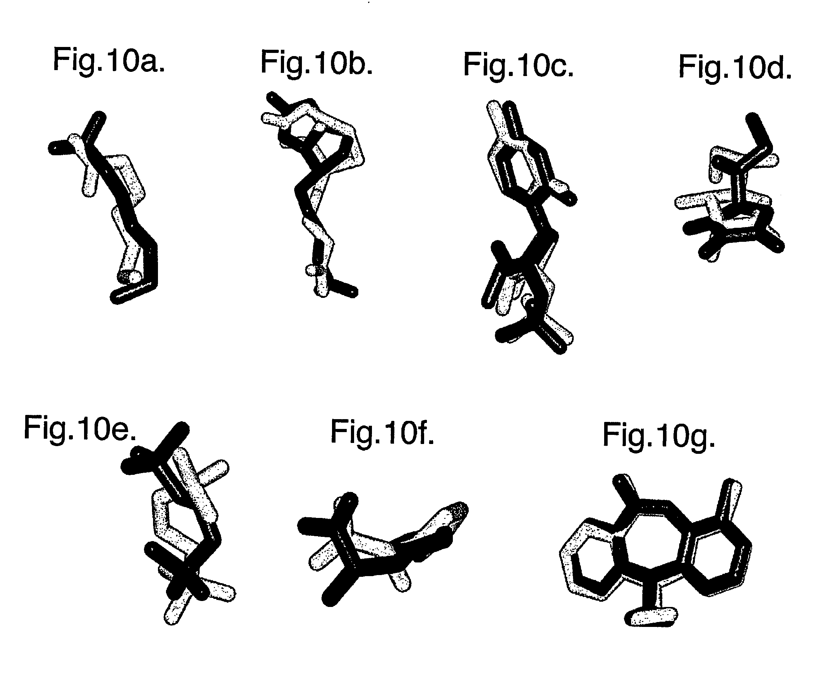

**Figure 10** shows a comparison between the further test results (shown as a light grey stick model) and the conformation of the ligand in the crystal structure (shown as a dark grey stick model) for the seven ligands: (a) $\varepsilon$-amino capronic acid, (b) biotin, (c) cytidylic acid, (d) L-ascorbic acid, (e) phosphocholine, (f) L-histidine, (g) nevirapin.

The *multi-scale approach* relies on a construct known as a *scale-space decomposition.* This involves the application of a *scaling operator* to the original data. The effect of the scaling operator is to remove the information in our data corresponding to the highest level of detail. With the fine detail removed, the larger scale features in the data are emphasised. Repeated application of the scaling operator returns larger and larger structures in our data until either the required scale has been achieved or there is no more information left in the resulting decomposition. The selection of the scaling operator is of crucial importance if multi-scale analysis via *scale-space decomposition* is to be completed successfully. According to the present embodiment of the invention, most of the information on the ligand is removed initially and is then gradually reintroduced in the reverse process to the above described decomposition.

We model the ligand at various scales by employing a simple yet powerful method: the *k-means-clustering algorithm* (see Hartigan, J. *Clustering Algorithms;* Wiley: New York, 1975, 84-112 and Rollet, R.; Benie, G. B.; Li, W.; Wang, S.; Boucher, J. M. *Int. J. Remote Sens.* **1998**, *19*, 3003-3009).

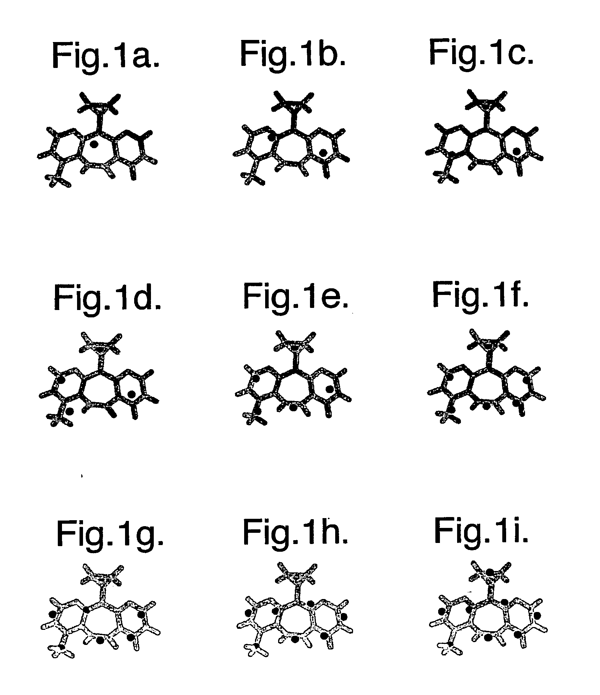

By using a series of *k-means* generated clusters we obtain series of ligand models, each containing one more feature point than the previous model. These feature points are well distributed to ensure that each model yields the best possible description of the ligand for the number of points generated. In our *k-means* procedure implementation, n atoms $x_1$, $x_2$, ..., $x_n$ fall into k clusters, $k < n$. Let $m_i$ be the mean position of the atomic coordinates in cluster i. If the clusters are well separated, we can use a minimum-distance classifier to separate them: atom $x$ is in cluster $i$ if the distance between $x$ and $m_i$ is the minimum of all the $k$ distances. Since there is no definite way to initialize the mean values, it is common to use random initial values for the means $m_1$, $m_2$, ..., $m_k$. At the next stage, until there are no changes in any mean, we use the estimated means to classify the atoms into clusters: for all clusters, replace $m_i$ with the mean position of all of the atoms for cluster *i*. Figure 1 depicts *k means* for an increasing number of feature points (from 1 to 9) on the HIV-Reverse Transcriptase inhibitor, nevirapine.

The initial cluster is at the mean position of the ligand: all of the atoms in the ligand belong to this initial cluster (Figure 1a). To initialize the means for the second cluster, we search for the atom that is furthest away from the initial cluster centre. This atom then becomes the temporary centre of the new cluster. All atoms that are closer to this cluster centre than their currently assigned cluster centre change identities and are marked as belonging to the new cluster. The position of the cluster centres are then iterated upon to self-consistency so that each cluster centre is positioned at the average position of the atoms that belong to the cluster (Figure 1b). This process may be repeated as many times as there are atoms in the ligand, with each iteration generating the next model (Figure 1c-i).

A rapid, grid-based method is employed for energy evaluation (see e.g. Wade, RC.; Goodford P.J. *J. Med. Chem.* **1993**, *36*, 140-147 and Wade, R.C, Goodford P.J. *J Med. Chem.* **1993**, *36*, 148-156) by pre-calculating ligand-protein pairwise interaction energies to form a lookup table. Energies are linearly interpolated from the grid. The energy is computed by equation 1 with the Consistent Valence Force Field (CVFF) (MSI, San Diego, CA) all-atom model nonbonding 12-6 Lennard-Jones and electrostatic terms. $A_{ij}$ is the repulsion parameter for the two ($i$, $j$) atoms, $B_{ij}$ is their attractive polarizability parameter, $q_i$ is the partial charge and ε is the dielectric constant Atom *i* belongs to a feature point $k$, and *j* is a protein grid point. It should be noted that the atoms' sum in all *k* feature points is identical to the number of atoms in the ligand.

The energy is calculated between all *i* atoms in feature point k and each protein atom (*j*). The process is repeated for all *k* feature points. The atom types in feature point *k* are different but their distances to protein's atom *j* are identical since they are calculated from feature point *k*.

$$[1] \qquad E_{pot} = \sum_k \sum_{i,j} \left( \frac{A_{ij}}{r_{kj}^{12}} - \frac{B_{ij}}{r_{kj}^{6}} + \frac{q_i q_j}{\varepsilon \, r_{kj}} \right)$$

An array of ligand models is generated in the manner described above. The first model, a single point, is then tested at all configurations in the host molecular field. Because a single point cannot be rotated, the test is extremely simple. All configurations whose energy is lower than a given threshold are marked to be kept for the next round of calculation. In the current implementation, configurations that do not demonstrate any avidity towards their binding site, i.e. their interaction energy is not negative, are evicted from subsequent iterations. Among the positions that are rejected from the next round are those that are either too far away or too close to the host molecule to be considered as likely docking configurations. Further, this simple test distinguishes between potentially good (negative interaction binding energy) or poor ligands. As more feature points are added the more implicit this classification becomes.

At each configuration that survived the first round (iteration) we test the second model. The second model is formed from two points separated by a certain distance that is related to the dimensions of the 'main axis' of the ligand. The orientation of the model is now important and consequently each configuration has to consider all possible rotations of the model. However, because the model contains only two points, the rotation is much faster than attempting to rotate the complete ligand. Once more, all configurations whose nonbonding energy is lower than a given threshold of the two models are kept for the next round. It is clear that this second model begins to filter out any configurations where the potential binding site is not large enough to hold a molecule as large as the ligand.

Repeating these steps for the subsequent models removes more of the unsuitable configurations. Eventually we will end up with only a few of the configurations surviving, usually long before we run out of models to test. These surviving configurations show the regions where it is possible for the ligand to dock successfully and the translation of the docked ligand.

In this context, the term configuration encompasses the spatial position and orientation of the ligand or ligand model relative to the protein or other macromolecule.

**Example Results**

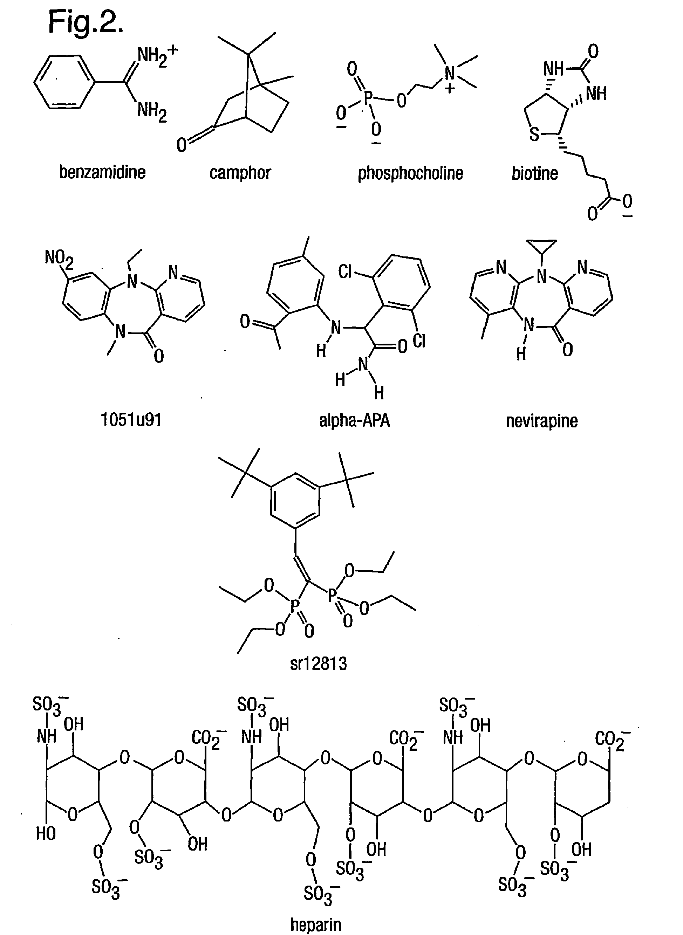

To test the methodology a number of host molecules complexed with ligands (Figure 2) were downloaded from the Protein Data Bank (PDB; see Berman, H.M.; Westbrook, J.; Feng, Z.; Gilliland, G.; Bhat, T.N.; Weissig, H.; Shindyalov, IN.; Bourne, P.E. *Nucleic Acids Res.* **2000**, *28*, 235-242). The ligands were deleted, and an attempt was then made to find the correct docking site of the ligands. In all test cases we placed the host protein in a box of dimensions 3Å greater in each direction than the extent of the protein. We employed a molecular grid with a 0.7Å resolution, and a rotation angle of 5°. A distance dependent dielectric constant of ε=4r was used.

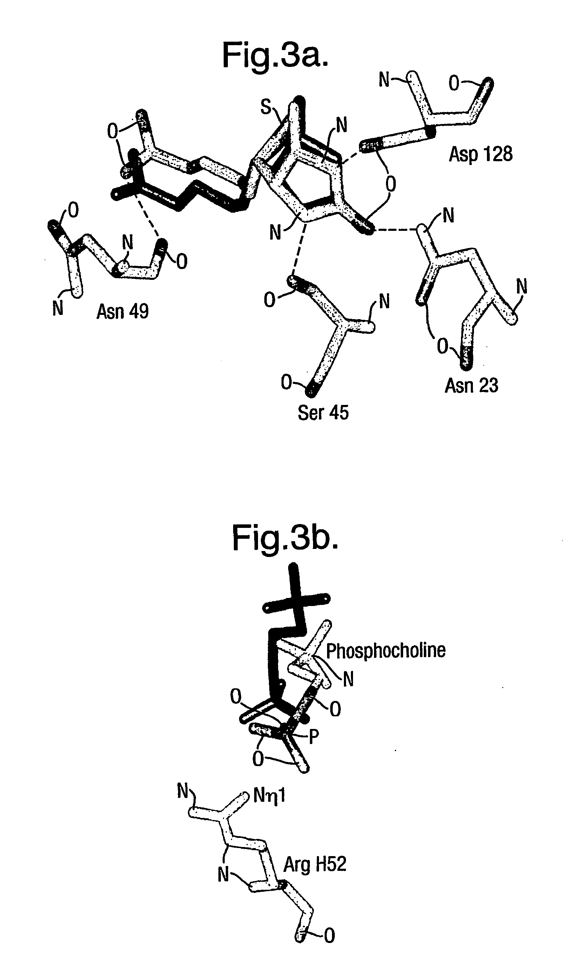

**Streptavidin/biotin.** We utilized the streptavidin complex with biotin (pdb entry 1stp; resolution 2.6Å). The search results are shown in Table 1 and Figure 3a. The distance between the centroid of biotin in the crystal structure and the centroid of its predicted configuration is 1.15Å. The multiple hydrogen bonds to N23, S45, N49, and D128, which are among the factors that allow a tight binding to the protein (shown by a dashed line), are presevered in our predicted configuration. The quality of the *population* of results was evaluated by calculating the distance between the centroid of the 5 lowest energy configurations and the centroid of the ligand in the crystal structure, which is 0.93Å in this case. Conjugate gradients local optimization was then employed on the complex with biotin is in its predicted position. The protein's atoms were held fixed and the ligand was allowed to move. The calculation converged after 356 iterations (12 seconds on R10000 single processor) when a convergence criterion of 0.01 kcal/Å had been achieved. The distance between the centroid of biotin in the crystal structure and the centroid of its predicted configuration after the minimization was reduced to 0.37Å.

**McPC-603/phosphocholine.** The Immunoglobulin McPC603 Fab-Phosphocholine complex was retrieved as pdb entry 2mcp. Since the force field was unable to assign reliable partial charges to the phosphocholine ligand, its partial charges were calculated with *Gaussian 98* program (Revision A.7) from Gaussian Inc, Pittsburgh PA, using a Hartree-Fock calculation with the STO-3G basis set. The distance between the centroid of phosphocholine in the crystal structure and the centroid of its predicted configuration is 2.02Å. The phosphocholine recognition by McPC-603 is predominantly electrostatic in character, primarily due to the influence of Arg H52. As can be seen from Figure 3b, the distance between the $N\eta1$ in the positively charged side chain of Arg H52 and the P atom in the negatively charged phosphocholine moiety is 3.12Å in the crystal structure, while in our predicted configuration this distance is 4.66Å. The distance between the centroid of the 5 lowest energy configurations and the centroid of the ligand in the crystal structure is 1.70Å. Employing the same minimization protocol as in the streptavidin/biotin test case, convergence was achieved after 159 iterations (17 seconds on R10000 single processor). The distance between the centroid of phosphocholine in the crystal structure and the centroid of its predicted configuration after the minimization was 1.90Å.

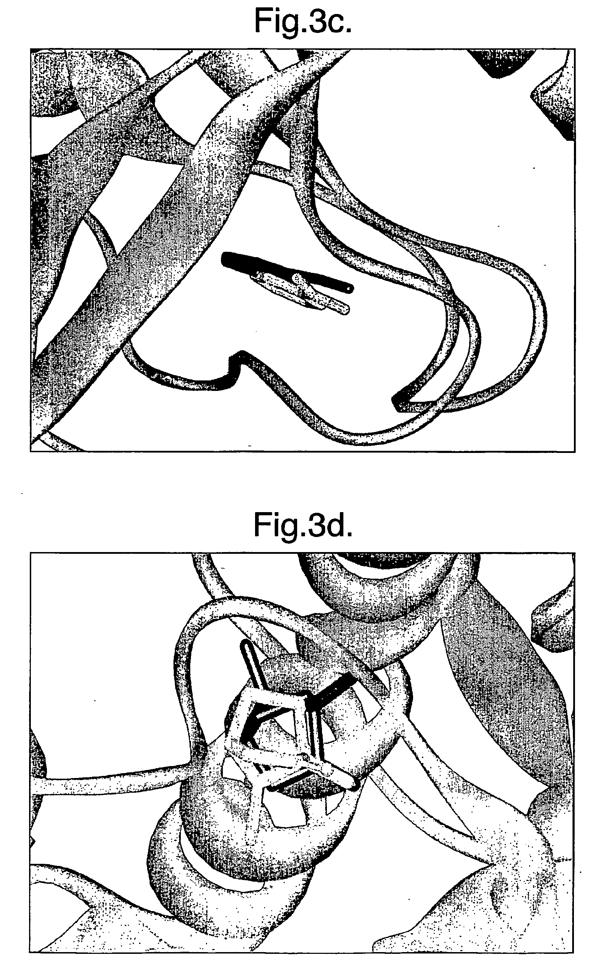

**β-Trypsin/benzamidine.** The Benzamidine Inhibited β-Trypsin was downloaded as pdb entry 3ptb. The distance between the centroid of biotin in the crystal structure and the centroid of its predicted configuration is 1.68Å. As can be seen from Figure 3c, the aromatic ring clearly fills the hydrophobic binding pocket. The distance between the centroid of the 5 lowest energy configurations and the centroid of the ligand in the crystal structure is 1.59Å. Again, the ligand was minimized and converged after 894 iterations (25 seconds on R10000 single processor). The distance between the centroid of benzamidine in the crystal structure and the centroid of its predicted configuration after the minimization was reduced to 0.38Å.

**Cytochrome P-450 cam/camphor.** The pdb entry 2cpp contains a protoporphyrin group with $Fe^{3+}$, and camphor. Because the force field did not contain proper partial charges for the protoporphyrin group with $Fe^{3+}$, the partial charges for this moiety were calculated as before. The distance between the centroid of camphor in the crystal structure and the centroid of its predicted configuration is 0.62Å. The distance between the centroid of the 5 lowest energy grid points and the centroid of the ligand in the crystal structure is 0.85Å. Figure 3d compares the low energy solution created by the algorithm to camphor in the crystal structure. The algorithm successfully detected the translation of the camphor, and the correct orientation of camphor was included in the low energy population but not ranked as the one with the lowest energy. We employed the same minimization protocol as in the streptavidin/biotin test case. The minimization converged after 677 iterations (55 seconds on R10000 single processor). The distance between the centroid of benzamidine in the crystal structure and the centroid of its predicted configuration after the minimization was reduced to 0.54Å.

**HIV-reverse transcriptase/Non-nucleoside inhibitors (NNIs).** We utilized the pdb file 1vrt (resolution 2.2Å) of HIV-reverse transcriptase as a host molecule for this test case and attempted to dock three non-nucleoside inhibitors (NNIs). The first inhibitor was nevirapine, which is complexed with the protein in this pdb entry. The second inhibitor was 1051U91 taken from the AZT resistant HIV-1 reverse transcriptase complex (pdb entry 1rt3), which included the mutations $D67 \rightarrow N$, $K70 \rightarrow R$, $T215 \rightarrow F$, $K219 \rightarrow Q$. The root mean square (RMS) deviation of the active sites (P95, L100, K101, V106, E138, V179, Y181, Y188, G190, F227, W229, L234, H235, Y318, a total number of 130 "heavy" atoms) in these two structures is 1.69Å. The high RMS value is due to Y181, which adopts a different rotamer in these structures. The third inhibitor was α-anilino phenyl acetamide (α-APA) taken from the pdb entry 1vru (resolution 2.4Å). The RMS of this active site's residues to the active site of 1vrt was 0.72Å.

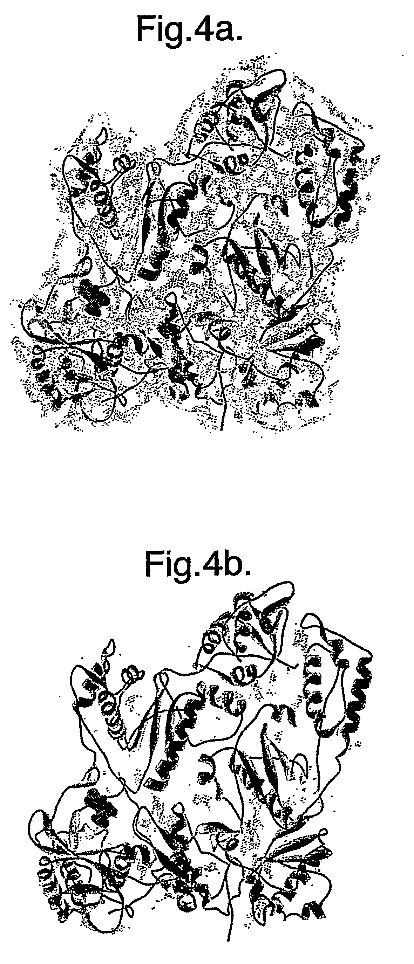

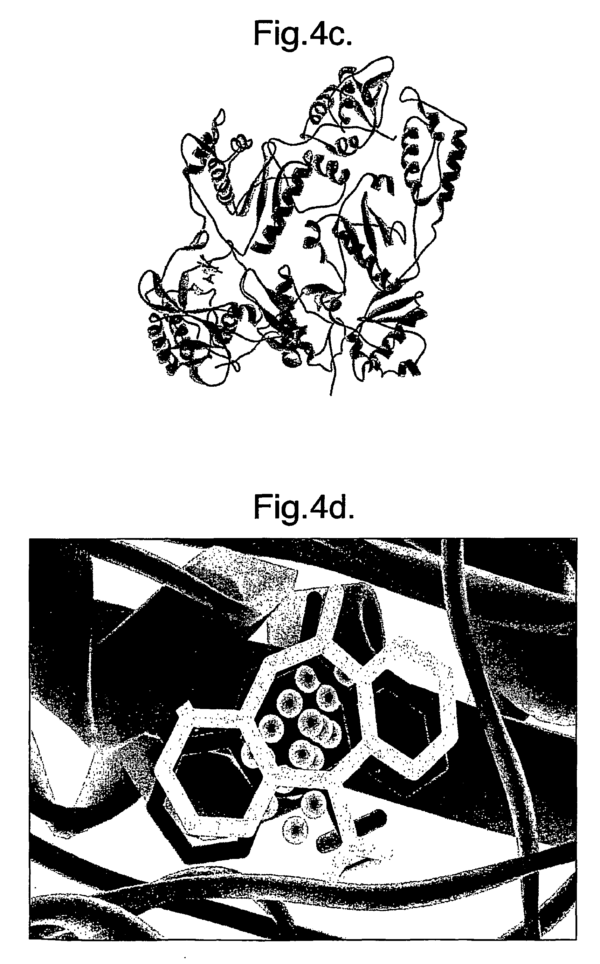

**Three docking stages for nevirapine are shown in Figure 4. The 20,000 lowest energy positions at the end of the first iteration are shown in Figure 4a. It can be seen that this population shows points either not too distant or too close to the protein. Figure 4b shows the points remaining after the third iteration. It can be seen that the remaining points converge to various pockets in the protein. Figure 4c shows the remaining points at the last iteration. Clearly, all points are focused in the actual binding site. The distances between the centroid of the ligand in the 1vrt crystal structure and the predicted configuration's centroid with the lowest energy is 1.15Å, 1.62Å and 1.20Å for nevirapine, 1051U91 and α-APA respectively. For the 5 lowest energy grid points' centroids this distance is 0.95Å, 0.90Å, 1.49Å for nevirapine, 1051U91 and α-APA respectively. Analysis of the interactions between the active site residues and the predicted positions of the inhibitors agrees with the results published by Ren et al. (Ren, J.; Esnouf, R.; Garman, E.; Somers, D.; Kirby, C. R I.; Keeling, J.; Darby, G.; Jones, Y.; Stuart, D. I.; Stammers, D. *Nat. Struct. Biol.* **1995**, *2*, 293-302) showing that all three inhibitors make extensive contacts (distance between "heavy atoms" <4.0Å) with Y181, 188 and Y318 as can be seen in Figure 4e (6 atoms for Nevirapine, 10 atoms for 1051U91, 8 atoms for α-APA). We employed the same minimization protocol as in the streptavidin/biotin test case. The minimization converged after 448 iterations (157 seconds on R10000 single processor). The distance between the centroid of nevirapine in the crystal structure and the centroid of its predicted configuration after the minimization was reduced to 0.32Å.

**Human nuclear pregnane X receptor (hPXR) - SR12813. The human** nuclear pregnane X receptor (hPXR) plays a critical role in mediating dangerous drug-drug interactions. Watkins et al. (Watkins, RE.; Wisely, G.B.; Moore, L.B.; Collins, J.L.; Lambert, M.H.; Williams, S.P.; Willson, T.M.; Kliewer, S.A.; Redinbo, M.R *Science* **2001**, *292*, 2329-2333) have recently solved the apo structure of the ligand-binding domain of hPXR and the complex with the cholesterol-lowering drug SR12813 at resolutions of 2.5Å (pdb file lilg) and 2.75Å (1ilh), respectively. Strikingly, SR12813 can bind in three distinct orientations. The distance between the centroids of the orientations ranges between 1.35-2.44Å. The RMS deviation between the binding site (L206, S208, L209, V211, L240, M243, M246, S247, F251, F281, C284, Q285, F288, W299, M323, L324, H407, R410, F420, a total number of 338 "heavy" atoms) in the apo and ligand-bound forms is 1.00Å. In order to test if the algorithm can handle protein plasticity, or a situation were the ligand is not well adapted, we employed our program on the apo structure and compared the binding results to the complexed one. The distances between the centroids of the three SR12813 orientations in the complex crystal structure and the centroid of its predicted configuration were 1.20Å, 2.05Å and 2.91Å respectively. Subsequent 701 iterations using the above minimzation protocol (147 seconds on R10000 single processor) reduced the distances to 0.65Å, 1.11Å and 1.82Å respectively.

**Heparin-basic fibroblast growth factor (bFGF).** This differs from the above test cases that involved small ligands, since heparin is a biomolecule (extents of 24.05Å x 13.68Å x 13.08Å, 152 atoms) and we were interested in a qualitative result. We utilized the pdb file 1bfc (2.2Å resolution) containing heparin hexamer fragment - basic fibroblast growth factor (bFGF) complex. Unlike most complexes that fit the standard 'lock and key' paradigm, with the ligand and host exhibiting high degree of surface complementarity, the heparin-binding site on bFGF is sterically ill defined. Instead it appears as if the ligand is held in place due to electrostatic interactions between the highly negatively charged heparin and its binding site. The calculation started with an initial number of 219,356 translations. The binding site was clearly identified (Figure 5).

Embodiments of the present invention provide a tool that is able to locate reliably the binding site for a specified host-ligand pair. As demonstrated by the results, the invention is extremely successful. In all quantitative test cases, except McPC-603/phosphocholine, the distance between the centroid of the ligand in the crystal structure and the centroid of its predicted configuration ranged from 0.62Å to 1.68Å. Bearing in mind that the resolution of the McPC-603/phosphocholine complex (2mcp) is only 3.1Å, this may hint that a distance of 2.02Å is a sensible result. In the case of hPXR/SR12813 the algorithm showed the best result for the first orientation (1.20Å) of the ligand. In addition it showed reasonable results for the second (2.05Å) and third (2.91Å) orientations as well.

Further, we have shown that not only the lowest energy solution but the *population* of the low energy solutions is very accurate. In all test cases the distance between the centroid of the ligand in the crystal structure and the centroid of its predicted configuration *population* ranged from 0.85Å to 1.70Å. On average, the *population* results were better than the lowest energy results. This fact suggests that the algorithm is consistent and converges to one site-the binding site.

The McPC-603/phosphocholine and HIV-reverse transcriptase/NNIs experiments represent a particularly harsh test of the technique. The phosphocholine ligand is extremely small (11 "heavy" atoms) relative to the size of the McPC-603 (442 residues, 3401 "heavy" atoms). As can be seen from the crystal structure data (pdb file 1vrt) and Figure 4, the HIV-reverse transcriptase host molecule is an extremely large molecule (926 amino acids, 7625 "heavy" atoms) compared to the three NNIs that we attempted to dock: 20, 22 and 21 "heavy" atoms for nevirapine, 1051U91 and α-APA respectively. Because of this disparity in size it is likely that the host will present many pockets into which the inhibitor could dock, in addition to the true binding site. α-APA and 1051U91 are difficult test cases since they are taken from a different configuration of the active site and their bioactive configuration, despite their relative rigidity, might vary to a certain degree among complexes. Further, unlike other test cases such as McPC-603/phosphocholine, the interactions between the host molecules and the ligand are hydrophobic and not electrostatic. Although we are comparing three different ligands, the small distances between the centroids in the predicted and the crystal structure demonstrate that the active site was successfully detected.

In addition to the translation, the orientation of the ligand in the binding site was found in all test cases. In 6 out of 8 it has been ranked as the one with the lowest energy. The first exception was hPXR/SR12813, which is an ambiguous test case, since there are three "correct" orientations. In camphor, the correct rotation was included in the low energy set of solutions but not ranked as the one with the lowest energy. These findings suggest further embodiments that employing more sophisticated cost functions than CVFF nonbonding energy terms, such as an empirical free energy function that estimates the free energy change upon binding or a finite difference Poisson-Boltzmann method to represent the electrostatic properties of the molecules to yield more accurate energies. In the embodiment used in the above examples we employ a linear interpolation approach to obtain the energies from the grid. Using a more sophisticated interpolation strategy could further improve the results.

The current implementation of the *multi-scale approach* is conducted in a discrete search space. As a result, the accuracy is limited both by the resolution of the grid and the size of the rotation angle. We have shown that our method produces an excellent starting point for rapid local optimization employed on a continuous search space using the same energy function. As demonstrated by the results, in all test cases, except McPC-603/phosphocholine, the distance between the centroid of the ligand in the crystal structure and the centroid of its predicted configuration after a short optimization ranged from 0.32Å to 0.65Å. Again, since the resolution of the McPC-603/phosphocholine complex (2mcp) is only 3.1Å we find a distance of 1.90Å as a reasonable result. We have shown that the minimization converges rapidly. Such results hint that the discrete search method we employ locates the ligand in close proximity to its "real" position and is reliable.

Most docking algorithms employ as test cases fixed structures of enzyme and/or ligand as taken from the enzyme-ligand complexes (pdb files). Here, one may raise the claim that there is a bias towards locating the true enzyme-ligand complex when scanning the interaction space. We have shown in two different test cases that the algorithm can cope with protein plasticity. In the HIV-reverse transcriptase/NNIs test case we have shown that we can successfully dock ligands taken from other crystal structures in a different bioactive configuration. In the hPXR-SR12813 system we successfully identified the binding site in the complexed protein where the input for our algorithm was the protein in its unbound configuration.

The invention offers several advantages over algorithms for detection of pockets on the surface of proteins such as LIGSITE. We have shown that instead of suggesting several binding pockets that exist in a large protein such as the HIV-reverse transcriptase, the algorithm embodying the invention is sensitive enough to detect the correct one. Further, it is sensitive enough to position the ligand in the correct binding orientation. The method performed well in the case of heparin-bFGF complex where there is a large ligand and no binding pocket.

The use of a *multi-scale approach* enables one efficiently to break a problem down into a number of small steps. Dismantling a problem in this manner enables efficient distribution of computing time so that only the most fruitful areas are considered in any detail. Embodiments of the invention have real value in exploiting the results emerging from studies in structural biology and can be a valuable tool to analyze the data from the structural genomics projects.

In the main embodiment described above, the interactions between the enzyme and the substrate are evaluated via simple nonbonding energy terms. Despite that, the results are accurate, and the calculations converges rapidly. This fact supports the ability of the *multi-scale approach* to represent the ligand in a reliable manner.

## Further embodiments of the invention

The above described embodiments and example results treated the ligand as a rigid molecule having a specific internal conformation, and identified an energetically favourable configuration of the ligand with respect to the macromolecule, that is the translational position of the centroid of the ligand and the rotational orientation of the ligand. The further embodiments and examples below extend the method of the invention to full flexible docking, that is to not only identifying the configuration (position and orientation) of the ligand with respect to the macromolecule when bound at a binding site, but also identifying the conformation of the ligand when bound, that is the arrangement of the atoms within the ligand molecule, including relative rotation of parts of the ligand about rotatable bonds. A conformation of the ligand is also referred to simply as a conformer.

First, we systematically create all the conformers for the ligand. The problem of adequate coverage of the conformational space in a reasonable computing time is also addressed *(vide infra):* only the torsion angles are modified, not the bond lengths or angles. Then, the conformers are ranked energetically. For this the Consistent Valence Force Field (CVFF) and all-atom model is employed. The conformers' energy is computed by equation 2 with the bonding 12-6 Lennard-Jones and electrostatic energy terms, where $A_{ij}$ is the repulsion parameter for the two $(i, j)$ atoms, $B_{ij}$ is their attractive polarizability parameter, $q_i$ is the partial charge, $r_{ij}$ is the distance between atoms, and $\varepsilon$ is the dielectric constant; $V_n$ is the torsional potential barrier height for a torsion angle $\Phi$, $n$ being the multiplicity and $\gamma$ the phase factor. If the energy term exceeds a given threshold, the conformer is ignored and will not be clustered or docked. At the end of this stage, we retain the population of lowest energy conformers.

$$[2] \quad E_{pot} = \sum_{i<j}(\frac{A_{ij}}{r_{ij}^{12}} - \frac{B_{ij}}{r_{ij}^{6}} + \frac{q_i q_j}{\varepsilon * r_{ij}}) + \sum_{dihedrals} \frac{V_n}{2}[1 + \cos(n\Phi - \gamma)]$$

To locate the biding site, we use a *multi-scale* concept where we deal with a hierarchy of models generated for the potential ligand, as previously described. Our search method is illustrated in figures 6 and 7. We model each conformer at various scales by employing the *k-means-clustering algorithm* as shown previously for rigid ligands. Each set conformer is assumed to be a single ligand. Figure 6 depicts *k-means* for an increasing number of feature points (from 1 to 3) on the HIV-Reverse Transcriptase inhibitor, nevirapine. Nevirapine has one rotatable bond (we ignore the torsion of the methyl moiety) as shown in figure 8g. If we systematically create all the conformers in 5° intervals, and assume all the conformers are energetically acceptable, we end up with n=72 conformers. In the first model, a single point, we end up with *n* points ($O_1$, $O_2$...$O_n$), as shown in figure 1. Clearly, all these points are identical and can be represented once instead of n times. The second model is two points separated by a certain distance ($a_1$, $a_2$...$a_n$) that is related to the dimensions of the 'main axis' of the conformer. In the case of nevirapine, the two-feature point representation has an average distance of 4.6 Å and minimal and maximal values of 4.3 Å and 4.9 Å respectively. Docking all the 72 clusters at this low level of detail is redundant. Instead, the *n*=72 clusters are purged by a *purge criterion* to *k* clusters, where $k \leq n$, and in most cases (see results) $k << n$. Instead of docking *n* clusters we can dock *k* clusters. A rather strict *purge criterion* of $\Delta a \leq 0.6$Å will yield a single representation in the case of nevirapine, i.e. from *n*=72 clusters, we simplified the problem to *k*=1 cluster. Indeed, at this level of representation and *purge criterion* the ligand is *treated* as one rigid cluster, although it is flexible. The third model is defined by two distances ($b_1$, $b_2$...$b_n$) and ($c_1$, $c_2$...$c_n$) separated by an angle ($\alpha_1$, $\alpha_2$...$\alpha_n$). It is purged into *p* presentations where the *purge criterion* comprises two distances and one angle. The number of three point *purged clusters* is equal or bigger than those for the two points representation, $k \leq p$. For the four-feature point representation, the purge criterion comprises three distances, two angles, and one dihedral. The process can be repeated for an increasing number of feature points.

The method of this embodiment of the invention builds a hierarchy tree of purged clusters as shown in figure 7. This figure describes a typical search tree for a flexible ligand. Each node contains one such purged cluster and a list of the ensemble of conformers this cluster stands for. In this example, two nodes of the purged clusters for two feature points emerge from the purged presentation of a single point. The method docks both of them, and proceeds with the node of lower energy, thus the docking procedure is only carried out along one branch of the tree of lowest energy, rather than for the whole tree. This process is repeated for the third point and so on. In this search only a small number of the purged representations will be docked. If we assume the four point representation is the last one to be docked, the worst case in this example is one rigid docking of a one point, two of two points, three of three points and five of four points. In the case illustrated in figure 7, for the nodes of three feature points, of the three cluster model nodes derived from the conformers represented by the lower energy node of two feature points, in the worst case scenario the middle one illustrated would have the lowest energy which would lead to five nodes of four feature points being required to be docked. These five nodes are obtained by: considering the conformers represented by that middle node of three feature points; representing each such conformer as a cluster of four feature points; purging those clusters to obtain the five to be used for docking.

The translation space is searched in a manner identical to that described previously. The only difference is the rapid tree search for each translation. The search begins from the single point representation. At the end of this stage, the method moves on the branch towards the two point and docks the purged representation that evolved from this single point. The process is repeated for the next purged representation where the parent of this representation is the one with the most favorable interaction energy with the binding site. At the last stage we employ a rapid, local optimization to refine the structure using the complete conformer with the binding site held fixed.

A grid-based method is employed for energy evaluation between the purged representation and the binding site by pre-calculating ligand-protein pairwise interaction energies to form a lookup table, as described previously with reference to equation 1.

In summary, the method according to the invention for taking into account the flexibility of the ligand to find an energetically favourable conformation of the ligand when bound, as well as assisting in finding an optimal binding site is as follows:

generate a representative set of conformations of the ligand, such as by rotating each rotatable bond through a predetermined angular interval;

evaluate the molecular energy of each conformation;

eliminate all conformations above a predetermined energy threshold;

represent each remaining conformation as a model (or cluster) with a number of feature points;

purge the models representing the conformations, using a purge criterion, to obtain a reduced number of representative models, each model representing an ensemble of conformations;

dock each representative model, for example using the methodology explained for the first aspect of the invention;

consider the ensemble of conformations represented only by the model with the most energetically favourable docking, eliminate the other conformation, and represent each remaining conformation as a model with an increased number of feature points from the previous iteration;

iterating by returning to the purging step, and terminating after a preset number of iterations or when a docking criterion has been met.

**Further Example Results**

To test the methodology, a number of protein ligand complexes were downloaded from the Protein Data Bank (PDB). The proteins chosen cover a range of sizes (80 to 926 residues), resolutions (1.6 Å to 3.1 Å) and fold families as shown in table 2 and figure 9. These proteins were: hydrolase (serine protease) (PDB entry 2pk4, figure 9a), streptavidin (1stp, 9b), hydrolase (endoribonuclease) (1rob, 9c), isomerase (1xid, 9d), McPC-603 (2mcp, 9e), histidine binding protein (1hsl, 9f) and HIV reverse transcriptase (1vrt, 9g). All ligands where flexible, with number of rotatable bonds ranging from 1 to 5, and number of atoms varying from 24 to 34 as shown in table 2 and figure 8.

We assumed the harshest situation where both the binding sites and the bioactive conformations of the ligand are unknown. The ligands were deleted, and an attempt was then made to find the correct docking site and the bioactive conformation of the ligands in one simulation. In all test cases we placed the host protein in a box of dimensions 3 Å greater in each direction than the extent of the protein. We employed a molecular grid with a 0.7 Å resolution, and a rotation angle of 5°. A distance dependent dielectric constant of $\varepsilon$=4r was used. We restricted the maximal representation of the ligands to 4 feature points. All conformers were systematically generated in 30° intervals. The question of whether the assumption of creating the conformers in 30° intervals is justified is addressed below. If the number of conformers exceeded the threshold of 1,000,000 then 1,000,0000 conformers were sampled at random. The lowest energy population of conformers (up to a threshold of 12 Kcal/mol) conformers was clustered. The clusters were purged using the following thresholds: distance < 1.3 Å, angle < 30°, and dihedral < 30°. Conjugate gradients local optimization was then employed on the complex from the starting geometry of the ligand in its predicted position. The protein's atoms were held fixed and the ligand was allowed to move until a convergence criterion of 0.01 kcal/Å had been achieved.

The search results are shown in table 2 and figures 9 and 10. The initial number of translations varied according to the size of the protein: from 151,156 for the smallest one, hydrolase (serine protease) (figure 9a), up to 2,666,664 translations for HIV reverse transcriptase (figure 9g). The Root Mean Square Deviation (RMSD) values when comparing our results to the crystal structure ranged from 0.29 Å (HIV-Reverse Transcriptase/nevirapineapin, figure 5g) to 2.43 Å (hydrolase/$\varepsilon$-amino capronic acid, figure 5a) with average value of 1.60 Å.

Figure 9 is a snapshot into the end of the third iteration. Even at this low level of detail, the calculation clearly converges to the binding site. In hydrolase (serine protease) (figure 9a), streptavidin (9b), hydrolase (endoribonuclease) (9c) and HIV reverse transcriptase (9g) all the remaining translations, even those with a low energetic score that were not yet evicted, fall into the binding site. In isomerase (9d) and McPC-603 (9e) the majority of translations, including those with the highest score (data not shown) fall into the binding site. The histidine binding protein/L-histidine complex (9f) was deposited in the Protein Data Bank as a dimer where L-histidine is bound to each of the monomers. We challenged the methodology and provided as an input the dimer and not a monomer. Both binding sites have been identified and ranked as the binding points with the lowest and same energy. This trend is suggested in figure 9f, where at the end of the third iteration most of the feature points already converge into the two binding sites.

The fact that from 1 up to 4 feature points are coarse representations of the ligand raises the issue of whether the method can use conformers created in larger dihedral angle intervals than 10° and still retain a reasonable coverage of all conformational space. Table 3 compares the computing time ratios needed to create conformers in 10° and 30° intervals. Biotin has $(360°/10°)^5$=60,466,176 conformers if we rotate in 10° intervals. If we change the 10° to 30°, the number of conformers will be $(360°/30°)^5$=248,832. In other words, we gain 60,466,176/248,832=243 times speedup. In the seven ligands we tested, the speedup varies from 3 for nevirapin up to 243 times for biotin and $\varepsilon$-amino capronic acid. We systematically created the conformers in 10° and 30° for the seven ligands, clustered all the conformers and purged the clusters using the same criteria of distance < 1.3Å, angle < 30° and dihedral < 30° and compared lowest 12 Kcal/mol populations. Table 3 shows the number of purged clusters needed to represent all the conformers of the seven ligands. At one feature point, it does not matter how flexible the ligand is, or by what increment we rotate the dihedral angles. In the case of two feature points, all ligands' conformations were still represented by one purged cluster, except biotin, which was represented by two. In other words, at this level of representation, all the conformational space of the ligands was compressed into one rigid line, except biotin, which was branched into two families. When we increase the level of detail to three and four feature points, more purged clusters are needed in order to represent the available conformational space. In the four point representation, the purged clusters number ranges from 1 for nevirapin up to 22 for biotin. This number is extremely small compared to the real number of conformers: 36 and 60,466,176 respectively (when creating the conformers in 10° intervals). When comparing the number of purged clusters when working in 10° and 30° intervals, in the case of one and two feature points, we got an identical number. This trend is preserved when moving to three feature points in 6 out of 7 ligands. The only exception, L-histidine, where there are 2 purged clusters in the 30° intervals comparing two three, in the case of 10°. When we move to a four feature point representation, 6 out of 7 ligands show 2/3 or more of the number of purged clusters when comparing the use of 10° and 30° increments.

Although an angular interval of 30° was used in the above examples for generating the conformers, the invention is not limited to that value, and successful results could be obtained using a larger angle, such as 45° or even 60°.

This further aspect of the invention provides a robust flexible docking methodology to proteins with unknown binding site or function. The approach confirms three assumptions. First, that it is possible to discretise the search space both for the translation, rotation and the conformational flexibility of the ligand. Second, we can search this space at various levels of detail using a multi-scale approach. Third, the result that emerges from this search is close enough to the optimal position, that by employing a rapid local optimization we can get a reliable prediction. The method is robust enough to find the binding site and the geometry of seven flexible ligands using only two basic assumptions. First, the protein's 3D structure is known; second, we have a reasonable "cost function", in this case a molecular mechanics based one.

An advantage of the method is that even if there is more than one binding site, such in the case of the histidine binding protein/L-histidine complex, one still needs only one simulation. It is impressive to see that both binding sites were identified and were ranked by identical energies (data not shown). Further, the ligand itself was docked with a reasonable degree of accuracy.

There was no correlation between the size of the protein (reflected by the initial number of translations) and the accuracy of the prediction. Strikingly, in the largest complex (HIV reverse transcriptase/nevirapin) we obtained the best prediction while in the smallest one (hydrolase/$\varepsilon$-amino capronic acid), we had the highest RMSD value. Hence, the quality of the prediction (but not the complexity of the problem) is not correlated to the number of initial translations. There was no correlation between the size of the ligand and the quality of the prediction as well. Indeed, the ligands with more than 30 atoms exhibited lower RMSD values than the ligands that had less than 30 atoms. The resolution of the crystal structure was not correlated to the quality of the prediction.

There is a weak correlation between the flexibility of the ligand and the quality of the predictions. Nevirapin, which was the least flexible ligand showed the lowest RMSD value, while $\varepsilon$-amino capronic acid, which has 5 rotatable bonds, showed the highest RMSD value. On the other hand, ligands such as cytidylic acid with 4 rotatable bonds showed lower RMSD than ligands with 2 rotatable bonds such as L-ascorbic acid. This indicates that there are other factors that affect the accuracy of the prediction. Three of them are discussed below.

First: the level of detail when discretizing the search space. Increasing this level, for example, by rotating the ligand in smaller increments or using a higher grid resolution than 0.7Å would improve the results.

Second, the detail level when using a multi-scale approach can effect the results. The maximum number of 4 feature points has only been used an example which provided good test case results. The optimal number of feature points can be determined as necessary. Large ligands may need more feature points. On the other hand, addition of feature points increases the ligand's detail level, hence the angle intervals that the ligand should be rotated should be smaller, otherwise many translations that are close to potentially good locations will not be found due to clashes with the protein. The sensitivity of the purge criterion is also crucial to the success of the method. Employing a loose criterion will lead to a small number of purged clusters (and nodes on the search tree) and will significantly reduce the computing time. However, such representation might be too coarse and consequently skew the results. On the other hand, employing a strict purge criterion will lead to a large number of clusters. This detailed presentation of a plethora of conformers is indeed more accurate. However, it will increase the computational demands. At first glance, there seems no clear relation between the angle intervals by which the torsions are rotated when creating conformers, and the purge criterion. However, if we employ a loose purge criterion, there is no point in rotating the torsions in very small intervals to generate an immense number of conformers since all, or the majority of them will be purged.

Third, more accurate energy functions than simple molecular mechanics nonbonding and torsion terms may be employed. Such energy functions can take into account other parameters such as conformational entropy loss due to the binding. Clearly, once the active site location is known, a computationally more expensive but accurate calculation can be repeated. The examples given simply demonstrate that by employing a simple and general energy function, such as CVFF, a multi-scale based flexible docking methodology is viable.

The methodology of this invention saves a substantial amount of computing time in several ways. First, it evicts at a very early stage all the unfavorable translations with a negligible computing time. Second, it uses a very small number of feature points in order to dock the seven ligands. Indeed, in this work, we clustered into 4 feature points seven ligands with a number of atoms that ranges from 24 to 34. Further, most of the translations are already evicted in 1-3 feature points in a less expensive computing time.

The methodology "rigidifies" the ligands. We are able to compress all the conformers into a small number of rigid feature points. This allows us to simplify the flexibility problem greatly. The most extreme example is nevirapin, which although flexible, was able to be treated as rigid during the docking. In 6 out of 7 ligands we showed that in the two point representation the flexibility problem can be is ignored.

Unlike other docking methods that normally rotate the ligand in 10°-15° intervals, here we employ much larger increments of 30°. We have shown that such increments are justified when using a multi-scale approach. In addition, we have shown a speedup that ranges from 3 to 243 times. In docking and in HTD, generating the conformers, storing them and later evaluating them is one of the bottlenecks. The problem of highly flexible compounds with prohibitively large number of conformers that cannot be evaluated exhaustively is a major one in chemoinformatics. In the anchor-first procedure (see Leach, A.R.; Kuntz, I.D. Conformational Analysis of Flexible Ligands in Macromolecular Receptor Sites. *J. Comput. Chem.* **1992**, *13*, 730-748), a rigid core is docked and the flexible parts reattached incrementally. There is a degree of bias in such a method since the geometry of the conformer in the binding site depends on the placement of the anchor. This is a limitation if the position of a ligand is dictated by a small functional group such as ammonium or carboyxlate, or if the ligand is aliphatic. Other chemoinformatics algorithms such as Statistical Classification of Activities of Molecules for Pharmacophore Identification (SCAMPI) (Chen, X.; Rusinko, A. 3rd; Tropsha, A.; Young S.S. Automated pharmacophore identification for large chemical data sets. *J. Chem. Inf. Comput. Sci.* **1999**, *39*, 887-896) sample confomations randomly. The present invention provides an advantageous, third option: creating the conformers in larger intervals and clustering them. An advantage of this method is in many cases, a uniform, non random and unbiased coverage of the whole conformational space.

Table 1. Results for the protein ligand complexes

| protein | PDB code | resolution (Å) | ligand | number of atoms in ligand[a] | initial number of translations | distance[b] between ligand centroid in the crystal and | | |
|---|---|---|---|---|---|---|---|---|
| | | | | | | ligand centroid in the lowest energy configuration | the centroid of the five low energy configurations | ligand centroid in the lowest energy configuration followed by a local minimization[c] |
| streptavidin | lstp | 2.6 | biotin | 31 | 257,816 | 1.15 | 0.93 | 0.37 |
| McPG603 | 2mcp | 3.1 | phosphocholine | 24 | 984,528 | 2.02 | 1.70 | 1.90 |
| β-Trypsin | 3ptb | 1.7 | benzamidine | 18 | 353,920 | 1.68 | 1.59 | 0.38 |
| cytochrome P-450$_{cam}$ | 2cpp | 1.63 | camphor | 27 | 772,500 | 0.62 | 0.85 | 0.54 |
| HIV-Reverse Transcriptase | 1vrt | 2.2 | nevirapine | 34 | 2,666,664 | 1.15 | 0.95 | 0.32 |
| | 1rt3 | 3.0 | 1051U91 | 36 | 2,666,664 | 1.62 | 0.90 | - |
| Human nuclear pregnane X receptor[d] | 1vru | 2.4 | α-APA | 39 | 2,666,664 | 1.20 | 1.49 | - |
| | 1ilg | 2.5 | - | - | - | - | - | - |
| | 1ilh | 2.75 | SR12813[e] | 75 | 709,152 | 1.20 | - | 0.65 |

[a] includes hydrogens

[b] distances are given in Å

[c] conjugate gradients minimization until convergence criterion of 0.01 kcal/Å is achieved where the protein's atoms are held fixed

[d] ligand was docked to the apo-structure (lilg) and compared to the complexed one (1ilh)

[a] SR12813 can bind in three distinct orientations. Results are shown for the first one

EP 1 468 392 B1

**Table 2**. protein ligand chosen protein as test cases

| protein | PDB code | resolution (Å) | number of residues in protein | ligand | number of atoms in ligand[a] | number of rotatable bonds | initial number of translations | RMSD[b] between the lowest energy prediction and the crystal structure |
|---|---|---|---|---|---|---|---|---|
| hydrolase (serine protease) | 2pk4 | 2.25 | 80 | ε-amino capronic acid | 22 | 5 | 151, 156 | 2.43 |
| streptavidin | 1stp | 2.6 | 121 | biotin | 31 | 5 | 257,816 | 1.28 |
| hydrolase (endoribonuclease) | 1rob | 1.6 | 124 | cytidylic acid | 33 | 4 | 270,396 | 0.90 |
| isomerase | lxid | 1.70 | 387 | L-ascorbic acid | 22 | 2 | 960,492 | 2.12 |
| McPC-603 | 2mcp | 3.1 | 442 | phosphocholine | 24 | 4 | 984,528 | 2.25 |
| histidine binding protein | 1hsl | 1.89 | 476 | L-histidine | 20 | 3 | 1,236,235 | 1.92 |
| HIV-Reverse Transcriptase | 1vrt | 2.2 | 926 | nevirapine | 34 | 1 | 2,666,664 | 0.29 |

[a] including hydrogens.
[b] RMSD is given in Å and was calculated between all non hydrogen atoms of the ligands.

**Table 3.** Number of purged clusters when roatating in 10° and 30° intervals

| ligand | rotatable bonds | when rotating in 10° intervals | | | | | when rotating in 30° intervals | | | | | speedup |
|---|---|---|---|---|---|---|---|---|---|---|---|---|
| | | number of conformers | number of purged clusters for | | | | number of conformers | number of purged clusters for | | | | |
| | | | 1 point | 2 points | 3 points | 4 points | | 1 point | 2 points | 3 points | 4 points | |
| ε-amino capronic acid | 5 | 60,466,176 | 1 | | 2 | 4 | 248,832 | 1 | 1 | 2 | 3 | 243 |
| biotin | 5 | 60,466,176 | 1 | 2 | 7 | 22 | 248,832 | 1 | 2 | 7 | 21 | 243 |
| cytidylic acid | 4 | 1,679,616 | 1 | 1 | 1 | 4 | 20,736 | 1 | 1 | 1 | 3 | 81 |
| L-ascorbic acid | 2 | 1,296 | 1 | 1 | 2 | 6 | 144 | 1 | 1 | 2 | 4 | 9 |
| phosphocholine | 4 | 331,776 | 1 | 1 | 3 | 9 | 20,736 | 1 | 1 | 3 | 6 | 16 |
| L-histidine | 3 | 46,656 | 1 | 1 | 3 | 6 | 1,728 | 1 | 1 | 2 | 3 | 27 |
| nevirapine | 1 | 36 | 1 | 1 | 1 | 1 | 12 | 1 | 1 | 1 | 1 | 3 |

EP 1 468 392 B1

**Claims**

1. A computer-based method for identifying a binding site for a ligand on a macromolecule and the configuration of the ligand when bound, said method comprising using a multiscale approach to represent the ligand as a plurality of models having different levels of detail.

2. A method according to claim 1, wherein using said multiscale approach comprises the steps of:

   representing the ligand as a model comprising a number of feature points fewer than the number of atoms in the ligand;
   evaluating the interaction energy between the ligand model and the macromolecule for a plurality of spatial positions and/or orientations of the ligand model, as represented by the feature points, relative to the macromolecule;
   eliminating those spatial positions and/or orientations for which the interaction energy does not satisfy a predetermined criterion;
   iterating the above steps using an increased number of feature points for the ligand model at each successive iteration.

3. A method according to claim 2, wherein the ligand is represented as a single feature point for the first iteration.

4. A method according to claim 2 or 3, wherein the number of feature points in the model representing the ligand is increased by one at each iteration.

5. A method according to claim 2, 3 or 4, wherein said predetermined criterion in said eliminating step is one of:

   that the interaction energy is below a threshold value, such as zero;
   that the interaction energy is ranked in the lowest predetermined number of all the energies for that particular iteration, such as the lowest 5; or
   that the interaction energy is ranked in the lowest predetermined fraction of all the energies for that particular iteration, such as the lowest 10%.

6. A method according any one of the preceding claims, comprising applying a k-means-clustering algorithm to represent the ligand as a model.

7. A method according to any one of the preceding claims, wherein the models of the ligand comprise averages of the atomic parameters of the ligand, said parameters comprising the spatial position of the atoms and at least one atomic force field descriptor.

8. A method according to claim 7, wherein said at least one atomic force field descriptor comprises at least one selected from the group consisting of: atom type, charge and polarizability.

9. A method according to any one of the preceding claims, further comprising terminating the iterative process after one of:

   a predetermined number of iterations;
   the number of non-eliminated configurations of the ligand model is smaller than a predetermined number;
   the distribution of non-eliminated configurations of the ligand model is within a predetermined range.

10. A method according to claim 9, further comprising determining the optimal binding site and ligand configuration by: selecting either that configuration model with the lowest energy or the average configuration of a predetermined number of lowest ranked configuration models; and reconstructing the ligand configuration from the selected model.

11. A method according to claim 10, further comprising applying a local energy minimization to determine an optimized binding site and ligand configuration.

12. A method according to any one of the preceding claims when appendant to claim 2, further comprising the step of:

   initially identifying a representative set of conformations of the ligand.

**13.** A method according to claim 12, wherein the representative set of conformations of the ligand is obtained by rotating each rotatable bond of the ligand through a predetermined angular interval.

**14.** A method according to claim 13, wherein the predetermined angular interval is greater than 10°, preferably greater than 20°, most preferably at least 30°.

**15.** A method according to claim 12, 13 or 14, wherein, if the number of conformations exceeds a predetermined quantity, then that quantity of conformers are sampled at random.

**16.** A method according to claim 12, 13, 14 or 15, further comprising the steps of:

evaluating the molecular energy of each identified conformation; and
eliminating all conformations whose energy exceeds a predetermined amount.

**17.** A method according to any one of claims 12 to 16, further comprising the steps of, at each iteration:

representing each remaining ligand conformation as a model comprising a number of feature points; and
applying a purge criterion to the model representations to obtain a smaller number of models for evaluating their interaction energy with the macromolecule.

**18.** A method according to claim 17, wherein the purge criterion comprises:

treating as a single model those models for which the variation in distance between particular feature points is less than 1.3 Å, and the variation in angle between particular arms of the model is less than 30° (for models with 3 or more points), and the variation in a particular dihedral angle is less than 30° (for models with 4 or more points).

**19.** A method according to claim 17 or 18, further comprising the step of, when increasing the number of feature points for the ligand model, only considering those ligand conformations represented by the model from the preceding iteration which had the lowest interaction energy with the macromolecule.

**20.** A method according to any one of the preceding claims further comprising outputting, in digital form, information representing at least one selected from the group consisting of the identified binding site, the ligand configuration when bound, and the ligand conformation when bound.

**21.** A method comprising using information representing at least one selected from the group consisting of the identified binding site, the ligand configuration when bound, and the ligand conformation when bound, obtained according to the method of any one of the preceding claims, to design mutations of the macromolecule and/or variations in the ligand which either agonise or antagonise the binding of the ligand at the binding site.

**22.** A method of identifying pharmaceutically useful compounds comprising the steps of:

repeating the method of any one of the preceding claims for a plurality of different ligands separately on the same macromolecule;
comparing the identified binding site for each ligand with a known binding site; and
identifying as useful compounds those ligands for which the identified binding site matches the known binding site.

**23.** A system for determining a binding site for a ligand on a macromolecule and the configuration of the ligand when bound, comprising:

interface means adapted to receive atomic information on the ligand and macromolecule;
a module comprising means to use a multiscale approach to represent the ligand as a plurality of models having different levels of detail and means to perform interaction energy evaluations to eliminate unfavourable binding sites and configurations; and
interface means adapted to output information on the or each determined binding site and ligand configuration.

**24.** A system according to claim 23, comprising a further module for including identification of favourable conformations of the ligand when bound.

**25.** A system according to claim 23 or 24 arranged to perform the method of any one of claims 1 to 22.

**26.** A computer program comprising code means which, when executed by a data processing system, performs all the method steps of a method according to any one of claims 1 to 22.

**27.** A computer-readable storage medium having recorded thereon a computer program according to claim 26.

**Patentansprüche**

**1.** Rechnerbasiertes Verfahren zum Identifizieren einer Bindungsstelle für einen Liganden an einem Makromolekül und der Konfiguration des Liganden nach Binden, wobei das Verfahren das Verwenden eines Multiskalen-Ansatzes zum Darstellen des Liganden als mehrere Modelle mit verschiedenen Detailebenen umfasst.

**2.** Verfahren nach Anspruch 1, **dadurch gekennzeichnet, dass** das Verwenden des Multiskalen-Ansatzes folgende Schritte umfasst:

Darstellen des Liganden als Modell, das eine Anzahl von Merkmalspunkten umfasst, die geringer als die Anzahl von Atomen in dem Liganden ist;

Beurteilen der Wechselwirkungsenergie zwischen dem Ligandenmodell und dem Makromolekül für mehrere räumliche Positionen und/oder Ausrichtungen des durch die Merkmalspunkte dargestellten Ligandenmodells im Verhältnis zum Makromolekül;

Eliminieren der räumlichen Positionen und/oder Ausrichtungen, bei denen die Wechselwirkungsenergie nicht ein vorbestimmtes Kriterium erfüllt;

Iterieren der obigen Schritte mit Hilfe einer größeren Anzahl an Merkmalspunkten für das Ligandenmodell bei jeder folgenden Iteration.

**3.** Verfahren nach Anspruch 2, **dadurch gekennzeichnet, dass** der Ligand für die erste Iteration als einzelner Merkmalspunkt dargestellt wird.

**4.** Verfahren nach Anspruch 2 oder 3, **dadurch gekennzeichnet, dass** die Anzahl an Merkmalspunkten in dem den Liganden darstellenden Modell bei jeder Iteration um eins erhöht wird.

**5.** Verfahren nach Anspruch 2, 3 oder 4, **dadurch gekennzeichnet, dass** das vorbestimmte Kriterium bei dem Schritt des Eliminierens eines von folgenden ist:

dass die Wechselwirkungsenergie unter einem Grenzwert wie etwa Null liegt;

dass die Wechselwirkungsenergie in der niedrigsten vorbestimmten Anzahl aller Energien für diese bestimmte Iteration eingestuft wird, wie etwa 5; oder

dass die Wechselwirkungsenergie in dem niedrigsten vorbestimmten Bruchteil aller Energien für diese bestimmte Iteration eingestuft wird, wie etwa die niedrigsten 10%.

**6.** Verfahren nach einem der vorhergehenden Ansprüche, welches das Verwenden eines K-Means-Clustering-Algorithmus zum Darstellen des Liganden als Modell umfasst.

**7.** Verfahren nach einem der vorhergehenden Ansprüche, **dadurch gekennzeichnet, dass** die Modelle des Liganden Mittel der Atomparameter des Liganden umfassen, wobei die Parameter die räumliche Position der Atome und mindestens einen Deskriptor eines atomaren Kraftfelds umfassen.

**8.** Verfahren nach Anspruch 7, **dadurch gekennzeichnet, dass** der mindestens eine Deskriptor eines atomaren Kraftfelds mindestens eines gewählt aus der Gruppe bestehend aus Atomtyp, Ladung und Polarisierbarkeit umfasst.

**9.** Verfahren nach einem der vorhergehenden Ansprüche, welches weiterhin das Beenden des iterativen Prozesses nach einem von:

einer vorbestimmten Anzahl an Iterationen;

die Anzahl nicht eliminierter Konfigurationen des Ligandenmodells ist kleiner als eine vorbestimmte Anzahl;

die Verteilung nicht eliminierter Konfigurationen des Ligandenmodells liegt innerhalb eines vorbestimmten Bereichs
umfasst.

**10.** Verfahren nach Anspruch 9, welches weiterhin das Ermitteln der optimalen Bindungsstelle und Ligandenkonfiguration durch: Wählen entweder des Konfigurationsmodells mit der niedrigsten Energie oder der durchschnittlichen Konfiguration einer vorbestimmten Anzahl an niedrigst eingestuften Konfigurationsmodellen und Rekonstruieren der Ligandenkonfiguration aus dem gewählten Modell umfasst:

**11.** Verfahren nach Anspruch 10, welches weiterhin das Anwenden einer lokalen Energieminimierung zum Ermitteln einer optimierten Bindungsstelle und Ligandenkonfiguration umfasst.

**12.** Verfahren nach einem der vorhergehenden Ansprüche bei Abhängigkeit von Anspruch 2, welches weiterhin folgenden Schritt umfasst:

zuerst Identifizieren einer repräsentativen Gruppe von Konformationen des Liganden.

**13.** Verfahren nach Anspruch 12, **dadurch gekennzeichnet, dass** die repräsentative Gruppe von Konformationen des Liganden durch Drehen jeder drehbaren Bindung des Liganden um einen vorbestimmten Winkelintervall erhalten wird.

**14.** Verfahren nach Anspruch 13, **dadurch gekennzeichnet, dass** der vorbestimmte Winkelintervall größer als 10°, bevorzugt größer als 20°, am bevorzugtesten mindestens 30° ist.

**15.** Verfahren nach Anspruch 12, 13 oder 14, **dadurch gekennzeichnet, dass**, wenn die Anzahl an Konformationen eine vorbestimmte Menge übersteigt, dann die Menge der Konformere zufällig abgetastet wird.

**16.** Verfahren nach Anspruch 12, 13, 14 oder 15, welches weiter die folgenden Schritte umfasst:

Beurteilen der Molekularenergie jeder identifizierten Konformation; und
Eliminieren aller Konformationen, deren Energie einen vorbestimmten Betrag übersteigt.

**17.** Verfahren nach einem der Ansprüche 12 bis 16, welches weiterhin bei jeder Iteration folgende Schritte umfasst:

Darstellen jeder verbleibenden Ligandenkonformation als Modell, das eine Anzahl von Merkmalspunkten umfasst; und
Anlegen eines Bereinigungskriteriums an den Modelldarstellungen, um eine kleinere Anzahl an Modellen zum Beurteilen ihrer Wechselwirkungsenergie mit dem Makromolekül zu erhalten.

**18.** Verfahren nach Anspruch 17, **dadurch gekennzeichnet, dass** das Bereinigungskriterium umfasst: Behandeln der Modelle als einziges Modell, bei denen die Änderung des Abstands zwischen bestimmten Merkmalspunkten unter 1,3 Å liegt und die Änderung des Winkels zwischen bestimmten Armen des Modells unter 30° liegt (bei Modellen mit 3 oder mehr Punkten) und die Änderung eines bestimmten Diederwinkels unter 30° liegt (bei Modellen mit 4 oder mehr Punkten).

**19.** Verfahren nach Anspruch 17 oder 18, welches weiterhin bei Erhöhen der Anzahl an Merkmalspunkten für das Ligandenmodell den Schritt nur des Berücksichtigens solcher Ligandenkonformationen umfasst, die durch Modell aus der vorhergehenden Iteration, das die niedrigste Wechselwirkungsenergie mit dem Makromolekül hatte, dargestellt werden.

**20.** Verfahren nach einem der vorhergehenden Ansprüche, welches weiterhin das Ausgeben von Angaben in digitaler Form umfasst, die mindestens eines gewählt aus der Gruppe bestehend aus der identifizierten Bindungsstelle, der Ligandenkonfiguration nach Binden und der Ligandenkonformation nach Binden darstellen.

**21.** Verfahren, welches das Verwenden von Angaben umfasst, die mindestens eines gewählt aus der Gruppe bestehend aus der identifizierten Bindungsstelle, der Ligandenkonfiguration nach Binden und der Ligandenkonformation nach Binden, die nach dem Verfahren eines der vorhergehenden Ansprüche erhalten wurden, darstellen, um Mutationen des Makromoleküls und/oder von Veränderungen des Liganden zu entwickeln, die die Bindung des Liganden an der Bindungsstelle entweder bewirken oder ihr entgegenwirken.

**22.** Verfahren zum Identifizieren von pharmazeutisch brauchbaren Verbindungen, welches die folgenden Schritte umfasst:

Wiederholen des Verfahrens nach einem der vorhergehenden Schritte für mehrere verschiedene Liganden separat an dem gleichen Makromolekül;
Vergleichen der identifizierten Bindungsstelle für jeden Liganden mit einer bekannten Bindungsstelle; und
Identifizieren solcher Liganden als brauchbare Verbindungen, bei denen die identifizierte Bindungsstelle der bekannten Bindungsstelle entspricht.

**23.** System zum Ermitteln einer Bindungsstelle für einen Liganden an einem Makromolekül und der Konfiguration des Liganden nach Binden, welches umfasst:

dafür ausgelegtes Schnittstellenmittel, atomare Angaben zum Liganden und Makromolekül zu erhalten;
ein Modul mit Mitteln zum Verwenden eines Multiskalen-Ansatzes zum Darstellen des Liganden als mehrere Modelle mit unterschiedlichen Detailebenen sowie mit Mitteln zum Ausführen von Wechselwirkungsenergie-Beurteilungen zum Eliminieren ungünstiger Bindungsstellen und Konfigurationen; sowie
dafür ausgelegte Schnittstellenmittel, Angaben zu der oder jeder ermittelten Bindungsstelle und Ligandenkonfiguration auszugeben.

**24.** System nach Anspruch 23, welches ein weiteres Modul zum Aufnehmen der Identifizierung günstiger Konformationen des Liganden nach Binden umfasst.

**25.** System nach Anspruch 23 oder 24, das zum Ausführen des Verfahrens nach einem der Ansprüche 1 bis 22 ausgelegt ist.

**26.** Computerprogramm mit Code-Mitteln, das bei Ausführen durch eine Datenverarbeitungsanlage all die Verfahrensschritte eines Verfahrens nach einem der Ansprüche 1 bis 22 durchführt.

**27.** Maschinell lesbares Speichermedium mit einem darauf gespeicherten Computerprogramm nach Anspruch 26.


**Revendications**

**1.** Procédé informatisé pour identifier un site de liaison pour un ligand sur une macromolécule et la configuration du ligand une fois lié, ledit procédé comprenant l'utilisation d'une approche multi-échelle pour représenter le ligand comme une pluralité de modèles ayant différents niveaux de détail.

**2.** Procédé selon la revendication 1, dans lequel l'utilisation de ladite approche multi-échelle comprend les étapes consistant à :

représenter le ligand comme un modèle comprenant un nombre de points de caractéristique inférieur au nombre d'atomes dans le ligand ;
évaluer l'énergie d'interaction entre le modèle de ligand et la macromolécule pour une pluralité de positions dans l'espace et/ou d'orientations du modèle de ligand, comme représentées par les points de caractéristique, relativement à la macromolécule ;
éliminer les positions spatiales et/ou les orientations pour lesquelles l'énergie d'interaction ne satisfait pas un critère prédéterminé ;
réitérer les étapes ci-dessus en utilisant un nombre plus important de points de caractéristique pour le modèle de ligand à chaque itération successive.

**3.** Procédé selon la revendication 2, dans lequel le ligand est représenté sous forme d'un point de caractéristique unique pour la première itération.

**4.** Procédé selon la revendication 2 ou 3, dans lequel le nombre de points de caractéristique dans le modèle représentant le ligand est augmenté d'un à chaque itération.

**5.** Procédé selon la revendication 2, 3 ou 4, dans lequel ledit critère prédéterminé dans ladite étape d'élimination est un parmi les suivants :

l'énergie d'interaction est inférieure à une valeur seuil, par exemple zéro ;
l'énergie d'interaction est classée dans le nombre prédéterminé le plus bas de toutes les énergies pour cette itération particulière, comme les 5 plus bas ; ou
l'énergie d'interaction est classée dans la fraction prédéterminée la plus basse de toutes les énergies pour cette itération particulière, comme les 10 % les plus bas.

6. Procédé selon l'une quelconque des revendications précédentes, comprenant l'application d'un algorithme de regroupement par la méthode des K centroïdes pour représenter le ligand comme modèle.

7. Procédé selon l'une quelconque des revendications précédentes, dans lequel les modèles du ligand comprennent des moyennes des paramètres atomiques du ligand, lesdits paramètres comprenant la position spatiale des atomes et au moins un descripteur de champ de force atomique.

8. Procédé selon la revendication 7, dans lequel au moins un descripteur de champ de force atomique comprend au moins un descripteur choisi dans le groupe consistant en : type d'atome, charge et polarisabilité.

9. Procédé selon l'une quelconque des revendications précédentes, comprenant en outre la fin du processus itératif après l'un de :

nombre prédéterminé d'itérations ;
nombre de configurations non éliminées du modèle de ligand inférieur à un nombre prédéterminé ;
distribution des configurations non éliminées du modèle de ligand comprise dans une plage prédéterminée.

10. Procédé selon la revendication 9, comprenant en outre l'étape consistant à déterminer le site de liaison optimal et la configuration de ligand en: sélectionnant soit le modèle de configuration avec l'énergie la plus faible soit la configuration moyenne d'un nombre prédéterminé de modèles de configuration de rang le plus bas ; et en reconstruisant la configuration de ligand à partir du modèle choisi.

11. Procédé selon la revendication 10, comprenant en outre l'application d'une minimisation d'énergie locale pour déterminer un site de liaison optimisé et une configuration de ligand.

12. Procédé selon l'une quelconque des revendications précédentes lorsque rattachées à la revendication 2, comprenant en outre l'étape consistant à :

identifier initialement un ensemble représentatif de conformations du ligand.

13. Procédé selon la revendication 12, dans lequel l'ensemble représentatif de conformations du ligand est obtenu en faisant tourner chaque liaison rotative du ligand sur un intervalle angulaire prédéterminé.

14. Procédé selon la revendication 13, dans lequel l'intervalle angulaire prédéterminé est supérieur à 10 °, de préférence supérieur à 20 °, de manière préférée entre toutes d'au moins 30 °.

15. Procédé selon la revendication 12, 13 ou 14, dans lequel, si le nombre de conformations dépasse une quantité prédéterminée, cette quantité de conformères est échantillonnée de manière aléatoire.

16. Procédé selon la revendication 12, 13, 14 ou 15, comprenant en outre les étapes consistant à:

évaluer l'énergie moléculaire de chaque conformation identifiée ; et
éliminer toutes les configurations dont l'énergie dépasse une quantité prédéterminée.

17. Procédé selon l'une quelconque des revendications 12 à 16, comprenant en outre pour chaque itération les étapes consistant à :

représenter chaque conformation de ligand restante comme un modèle comprenant un nombre de points de caractéristique ; et
appliquer un critère de purge aux représentations du modèle pour obtenir un nombre plus restreint de modèles pour évaluer leur énergie d'interaction avec la macromolécule.

**18.** Procédé selon la revendication 17, dans lequel le critère de purge comprend : le traitement comme modèle unique ces modèles pour lesquels la variation de distance entre des points de caractéristique particuliers est inférieure à 1,3 Å, et la variation d'angle entre des bras particuliers du modèle est inférieure à 30 ° (pour les modèles à 3 points ou plus) et la variation d'un angle dièdre particulier est inférieure à 30 ° (pour les modèles à 4 points ou plus).

**19.** Procédé selon la revendication 17 ou 18, comprenant en outre l'étape consistant à, lorsqu'on augmente le nombre de points de caractéristique pour le modèle de ligand, ne tenir compte que ces conformations de ligand représentées par le modèle de l'itération précédente qui avaient l'énergie d'interaction la plus faible avec la macromolécule.

**20.** Procédé selon l'une quelconque des revendications précédentes comprenant en outre l'émission, sous forme numérique, d'informations représentant au moins une information choisie dans le groupe consistant en le site de liaison identifié, la configuration du ligand une fois lié et la conformation du ligand une fois lié.

**21.** Procédé comprenant l'utilisation d'informations représentant au moins une information choisie dans le groupe consistant en le site de liaison identifié, la configuration du ligand une fois lié, et la conformation du ligand une fois lié, obtenues selon le procédé selon l'une quelconque des revendications précédentes, pour concevoir des mutations de la macromolécule et/ou des variations du ligand qui entraînent l'agonisme ou l'antagonisme de la liaison du ligand au niveau du site de liaison.

**22.** Procédé pour identifier des composés utiles pharmaceutiquement comprenant les étapes consistant à :

> répéter le procédé selon l'une quelconque des revendications précédentes pour une pluralité de ligands différents séparément sur la même macromolécule ;
> comparer le site de liaison identifié pour chaque ligand avec un site de liaison identifié ;
> identifier comme composés utiles ces ligands pour lesquels le site de liaison identifié correspond au site de liaison connu.

**23.** Système pour déterminer un site de liaison pour un ligand sur une macromolécule et la configuration du ligand une fois lié, comprenant :

> un moyen d'interface adapté pour recevoir les informations atomiques concernant le ligand et la macromolécule ;
> un module comprenant des moyens pour utiliser une approche multi-échelle pour représenter le ligand comme une pluralité de modèles ayant différents niveaux de détail et des moyens pour réaliser des évaluations d'énergie d'interaction pour éliminer des sites de liaison et des configurations défavorables ; et
> un moyen d'interface adapté pour émettre des informations sur le ou sur chaque site de liaison déterminé et la configuration de ligand.

**24.** Système selon la revendication 23, comprenant un autre module pour inclure l'identification de conformations favorables du ligand lié.

**25.** Système selon la revendication 23 ou 24 arrangé pour réaliser le procédé selon l'une quelconque des revendications 1 à 22.

**26.** Logiciel comprenant un moyen de code qui, une fois exécuté par un système de traitement des données, réalise toutes les étapes de procédé d'un procédé selon l'une quelconque des revendications 1 à 22.

**27.** Support d'enregistrement informatisé sur lequel est enregistré un logiciel selon la revendication 26.

Fig.1a.

Fig.1b.

Fig.1c.

Fig.1d.

Fig.1e.

Fig.1f.

Fig.1g.

Fig.1h.

Fig.1i.

# Fig.2.

benzamidine

camphor

phosphocholine

biotine

1051u91

alpha-APA

nevirapine

sr12813

heparin

# Fig.3a.

# Fig.3b.

## Fig.3c.

## Fig.3d.

# Fig.4a.

# Fig.4b.

## Fig.4c.

## Fig.4d.

# Fig.4e.

# Fig.5.

Fig.6.

# Fig.7.

Number of feature points

Conformers 1

Conformers 2

Conformers n

Fig.8a.  Fig.8b.  Fig.8c.  Fig.8d.  Fig.8e.  Fig.8f.  Fig.8g.

Fig.9a.
Fig.9b.
Fig.9c.
Fig.9d.
Fig.9e.
Fig.9f.
Fig.9g.

Fig.10a.

Fig.10b.

Fig.10c.

Fig.10d.

Fig.10e.

Fig.10f.

Fig.10g.